# EUROPEAN PATENT APPLICATION

(11) **EP 4 331 569 A1**
(43) Date of publication of application: **06.03.2024**
(21) Application number: 22926620.0
(22) Date of filing: 09.05.2022
(51) Int. Cl.: A61K 9/00, A61K 31/44, A61K 47/34, A61K 47/38, A61P 35/00

(54) **SORAFENIB OR DONAFENIB ORAL PREPARATION WITH LOW DOSE AND HIGH DRUG EXPOSURE, AND APPLICATION THEREOF**

(30) Priority: 21.02.2022 WO PCT/CN2022/077001
(71) Applicant: Beijing Creatron Institute of Pharmaceutical Research Co., Ltd., Beijing 102200 (CN); Tianjin Creatron Biotechnology Co., Ltd., Tianjin 300457 (CN)
(72) Inventor: JIA, Huijuan, Beijing 102200 (CN); HOU, Xin, Tianjin 300457 (CN); LI, Yan, Tianjin 300457 (CN); ZHANG, Jiayan, Beijing 102200 (CN); CHEN, Shengye, Tianjin 300457 (CN); REN, Xiaohui, Beijing 102200 (CN)
(74) Representative: Prüfer & Partner mbB Patentanwälte · Rechtsanwälte
(86) International application number: PCT/CN2022/091593
(87) International publication number: WO 2023/155307

(57) **Abstract**

The present invention provides a sonfenib or donafenib (TKI) oral preparation, comprising a TKI molecular-level mixture and self-assembled polymer materials. The present application also provides an application of the TKI oral preparation. The two self-assembled polymer materials in the TKI oral preparation provided by the present application perform coordinated regulation with TKI guest molecules to reduce premature degradation or efflux of a drug caused by CYP3A4/A5, CYP2D6, CYP2C8 or P-gp of P450 enzymes in epithelial cells of the gastrointestinal tract mucosa during an absorption process of the oral preparation, and reduce decreased absorption caused by rapid packing after the drug is quickly dissolved out of a molecular-level composition. Therefore, the oral preparation can achieve the same curative effect at a lower dose, features lower Cₘₐₓ and AUC₀₋ₜ variations, reduces of the risk of being non-effective or toxic, and would not be affected by food; in addition, the oral preparation significantly reduces dose-related side effects, improves patient compliance, and has good physical and chemical stability.

## Description

This application claims the priority of Chinese Patent Application No. PCT/CN2022/077001, filed with the China National Intellectual Property Administration on February 21, 2022, and titled with "SORAFENIB OR DONAFENIB ORAL PREPARATION WITH LOW DOSE AND HIGH DRUG EXPOSURE, AND APPLICATION THEREOF", the disclosures of which are hereby incorporated by reference in entirety.

### FIELD

The present disclosure relates to the fields of pharmaceutical preparation technology and materials science, and in particular to a pharmaceutical composition at the molecular level which, after being released in the body, restructures a new self-assembly system with self-assembling polymer materials, breaking the molecular packing formed by the interaction between sorafenib or donafenib molecules, reducing the premature metabolism of drugs by CYP3A4/A5/CYP2/P-gp in the digestive tract, and decreasing the toxicity and side effects on the gastrointestinal tract before the drug is absorbed into the system, and a lower dose of oral preparations of sorafenib or donafenib is adopted to realize its therapeutic application.

### BACKGROUND

Nexavar Sorafenib Tosylate Tablets is the first oral multi-target tumor molecular targeting drug jointly developed by Bayer and Onyx for the treatment of advanced liver cancer, renal cell carcinoma and thyroid cancer. Since the drug was launched as the first-line drug for the systemic treatment of advanced hepatocellular carcinoma in 2007, the systemic treatment of hepatocellular carcinoma has changed significantly. Sorafenib can act on tumor cells and tumor blood vessels at the same time, and play a role in inhibiting tumor growth through mechanisms such as anti-cell proliferation, promoting apoptosis and inhibiting angiogenesis. On the one hand, sorafenib exerts an anti-cell proliferation effect by inhibiting c-Raf kinase and downstream signaling, hindering the phosphorylation process of MEK and ERK, and reducing the phosphorylation level of ERK; on the other hand, sorafenib plays an anti-angiogenic role by inhibiting vascular endothelial growth factor receptor-2 (VEGFR-2), vascular endothelial growth factor receptor-3 (VEGFR-3) and platelet-derived growth factor receptor-β (PDGFR-β) and inhibiting autophosphorylation of tyrosine kinase receptors. It has dual anti-tumor effects: it can not only directly inhibit the proliferation of tumor cells by blocking the RAF/MEK/ERK-mediated signaling pathway, but also block the formation of new blood vessels in tumors by inhibiting VEGFR and platelet-derived growth factor (PDGF) receptors; in addition, it plays a role in promoting cell apoptosis by inhibiting the phosphorylation process of eukaryotic initiation factor-4E (eIF4E) and downregulating the level of anti-apoptosis protein Mcl-1 in vivo.

Sorafenib is an oral inhibitor against many kinases of the bisarylurea class, with the chemical name 4-[4-[[4-chloro-3-(trifluoromethyl)phenyl]carbamoylamino]phenoxy]-N-methylpyridine-2-carboxamide and a relative molecular weight of 464.8. The active ingredient used in Nexavar Sorafenib Tablets is tosylate of sorafenib, and the molecular formula is C₂₁H₁₆ClF₃N₄O₃·C₇H₈O₃S, as shown in Formula (2). The structure of sorafenib is as follows:

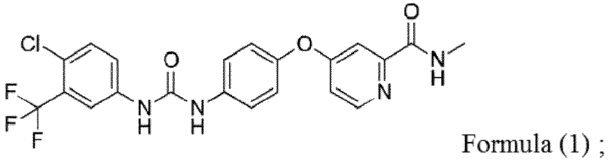

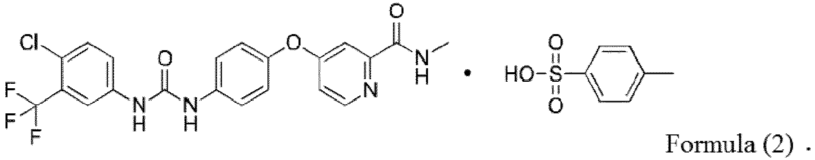

Donafenib is a deuterated drug of sorafenib developed by Suzhou Zelgen pharmaceuticals Co., Ltd., China. It has a chemical name 4-[4-[[4-chloro-3-(trifluoromethyl)phenyl]carbamoylamino]phenoxy]-N (trideuteriomethyl)pyridine-2-carboxamide, and its differences in chemical structure and physicochemical properties with those of sorafenib are shown in Table 1 below.

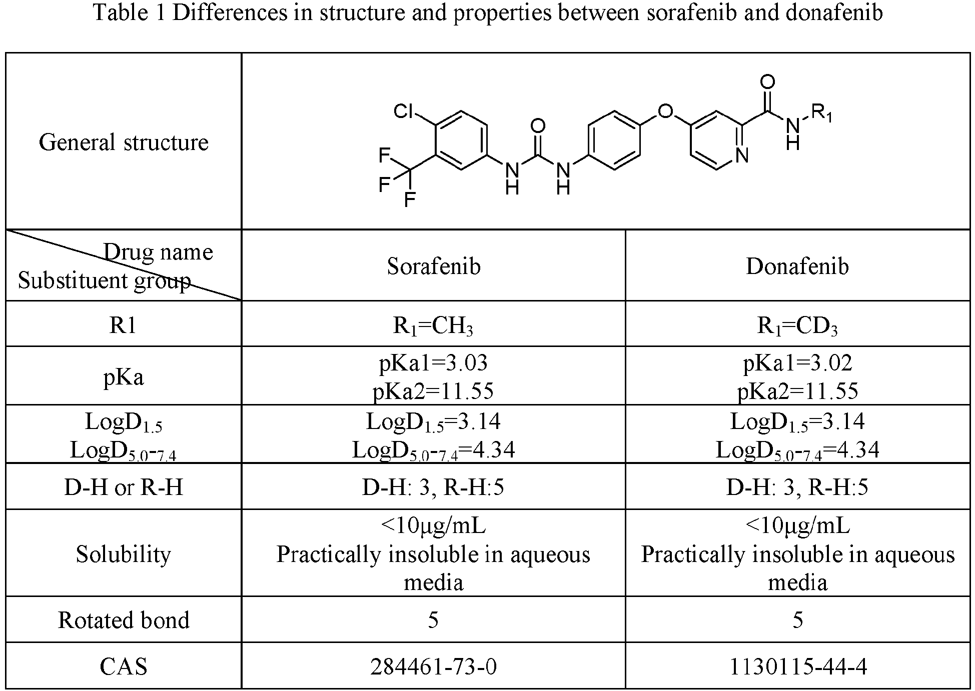

As can be seen from Table 1, the two are identical except for the methyl group linked by the amide bond. The deuterated group has no significant effects on intramolecular interactions, intermolecular interactions, pKa, hydrophobicity (LogP), the numbers of hydrogen donors, hydrogen acceptors and rotatable chemical bonds, and action sites of metabolic enzymes in vivo of the drug. As a result, except for the hydrogen methyl group or the deuterium methyl group in the structure, everything else is the same. Therefore, sorafenib is used as an example for further illustration.

Donafenib Tosylate Tablets was approved for marketing by China National Medical Products Administration in June 2021 as Class I innovative product. The marketed trade name is Zepsun, and the marketed dosage form is tablets with a specification of 100 mg, 2 tablets (200 mg) each time, twice a day, 400 mg.

According to the classification system of biopharmaceutics, sorafenib or donafenib are both BCS class IV drugs with low solubility and low permeability. Based on the instructions of Nexavar Sorafenib Tosylate Tablets, the average relative bioavailability of Sorafenib Tosylate Tablets is only 38%-49% compared to the oral solution. Therefore, the specification of Nexavar Sorafenib Tosylate Tablets is 200 mg, 2 tablets each time, twice a day, with the maximum daily dose of 800 mg. Based on the instructions of Nexavar Sorafenib Tablets, after oral administration of 100 mg of ¹⁴C radiolabeled sorafenib oral solution to healthy volunteers, 77% of the administered dose was excreted in feces, and the prototype drug accounted for more than 51% in feces, suggesting that one of the reasons for the low oral bioavailability of Nexavar Sorafenib Tosylate Tablets may be attributed to the solubility of the drug itself, as more than half of the administered dose of the drug was directly excreted in feces due to low solubility. The actual clinical dosage is 400 mg in a single dose, and yet there is currently no data on the amount of the drug prototype directly excreted through feces in the administered dose of 400 mg.

AAPS J. 2019 Jan 9, 21(2): 15, AAPS J. 2019 Oct 21;21(6): 107, Chem Biol Interact. 2021 Apr 1;338:109401, Xenobiotica 2016 Jul; 46(7): 651-658 and the instructions ofNexavar Sorafenib Tosylate Tablets indicate that the metabolism of sorafenib is mediated by CYP3A4/A5 and CYP2C8/D6 of P450 enzymes, and its metabolite is sorafenib-N-oxide. Sorafenib is also the substrate of efflux pump P-gp. These CYP3A4/A5, CYP2C8/D enzymes and P-gp are widely distributed in human intestinal epithelial cells, which is another main reason for the high variability after absorption in vivo and low oral bioavailability of sorafenib. Clinical trial results of sorafenib disclosed in Clin Cancer Res 2005 show that the pharmacokinetic parameters of sorafenib in humans have high individual variability that oral administration of 200 mg (Bid) or 400 mg (Bid) for 7 consecutive days presents coefficients of variation of Cₘₐₓ and AUC₀₋₁₂ₕ at steady state of 8.6% and 16.9% for 200 mg (Bid) and 106.7% and 90.5% for 400 mg (Bid). Therefore, for poorly soluble drugs, the patient's digestive environment (pH, food, enzymes and the amount of digestive juice) and the process of drugs entering the intestine from the stomach after oral administration have great influence on the absorption of sorafenib. Clinical high-dose administration may compensate for the high PK variation caused by P450 enzymes, but it will not compensate for the high PK variation caused by solubility problem. Oncotarget, 2017, Vol. 8, (26), pp: 43458-43469 discloses a clinical trial conducted on 94 patients with renal cell carcinoma, in which group 1 (n=74) was orally administered 400 mg sorafenib tablets (Bid), group 2 (n=18) was orally administered 600 mg sorafenib tablets (Tid), and group 3 (n=2) was orally administered 400 mg sorafenib tablets (Qd). After 2 weeks of treatment, the steady state concentration of sorafenib in the blood (plasma) was 881-12,526 ng/mL, and the major adverse reactions and their incidences were as follows: diarrhea (76.5%), hand-foot syndrome (68.99%) and fatigue (55.32%), all of which were related to the concentration of sorafenib in the blood. The blood drug concentration of patients who experienced serious adverse reactions exceeded 10,000 ng/ml, and these serious adverse reactions will be reduced with the decrease of dosage or discontinuation of the drug. This demonstrates that these serious side effects are all related to the administration dosage. Cancer Chemotherapy and Pharmacology (2020) 86:129-139 studied the effect of systemic exposure amount of sorafenib on the response, efficacy and safety of patients with advanced hepatocellular carcinoma. The results of the study show that a significant increase in grade 3 and 4 serious adverse reactions was reported for sorafenib tosylate tablets administered at a dose of 0.4 g (twice a day) compared with that administered at a dose of 0.2 g (twice a day), and many of grade 3 and 4 serious adverse reactions were significantly correlated with the trough concentration C_{trough} of sorafenib. When the trough concentration of sorafenib exceeded 3.45 µg/ml, the incidence of serious adverse reactions above grade 3 (elevated ALT, elevated AST, elevated esterase, hand-foot syndrome, hypertension, etc.) was 72.7%, and when the trough concentration of sorafenib was below 3.45 µg/ml, the incidence of serious adverse reactions above grade 3 was 26.7%. The high PK variation of Sorafenib Tosylate Tablets has a significant impact on the efficacy (time to treatment failure (TTF), progression-free survival (PFS) and overall survival (OS)). When the trough concentration of sorafenib <1.40 µg/mL, TTF, PFS and OS were 4.7 months, 4.7 months and 5.3 months respectively; when 1.40 µg/mL ≤sorafenib C_{trough} <3.45 µg/mL, TTF, PFS and OS were 13.1 months, 15.8 months and 17.8 months respectively (TTF, PFS and OS of Lenvatinib Mesilate Capsules (LENVIMA) were 8.9 months, 7.4 months and 13.6 months respectively); when the trough concentration of sorafenib ≥3.45 µg/mL, TTF, PFS and OS were 2.4 months, 8.9 months and 9.5 months respectively. Long-term sub-therapeutic concentrations (trough concentration below 1.40 µg/mL) will lead to poor therapeutic response and induce drug resistance, which is a common cause of cancer chemotherapy failure, while excessive therapeutic concentration (≥3.45 µg/mL) will lead to the multiplication of adverse reactions, and treatment was eventually abandoned due to intolerance of severe adverse reactions (TTF was 2.4 months), with a significant decrease in the overall survival (OS) and progression-free survival.

The above research results indicate that the reduction of high drug PK variability in treatment with sorafenib has great significance to reducing severe toxicity and ineffectiveness. Therefore, the development of a new generation of sorafenib therapeutic drug with low PK variability is of great clinical value. The prior art has conducted a lot of experimental studies and challenges in order to improve the oral bioavailability of sorafenib preparations, reduce the clinical dosage, and solve the thorny world problems such as high PK variability, but the core patent of Nexavar Sorafenib Tosylate Tablets has expired so far, and no new product that surpasses the existing listed drugs has yet been commercialized.

US20090203709 discloses a pharmaceutical dosage form comprising a solid dispersion product consisting of at least one tyrosine kinase inhibitor, at least one pharmaceutically acceptable polymer and at least one pharmaceutically acceptable solubilizer, wherein, the solubilizer is selected from the group consisting of polyol fatty acid esters, polyalkoxylated polyol fatty acid esters, polyalkoxylated fatty alcohol ether, tocopherol compound and mixtures thereof; and also discloses a method for preparing the above pharmaceutical dosage form, comprising preparing a homogeneous melt of at least one tyrosine kinase inhibitor, at least one pharmaceutically acceptable polymer and at least one pharmaceutically acceptable solubilizer, and then allowing the melt to solidify to obtain a solid dispersion product.

CN107115317 discloses a pharmaceutical composition comprising sorafenib, at least one high molecular polymer and a solubilizer to form amorphous hybrid nanoparticles; the composition further comprises at least one pharmaceutically acceptable solubilizer, which is selected from the group consisting of polyvinylcaprolactam-polyvinyl acetate-polyethylene glycol copolymer, d-α-tocopherol polyethylene glycol 1000 succinate (TPGS1000) and hydrogenated castor oil such as PEG-40 hydrogenated castor oil or PEG-35 hydrogenated castor oil. It also discloses a preparation method for producing the above pharmaceutical composition is supercritical fluid method.

WO2006026501A1 discloses a composition comprising sorafenib and/or its salts, hydrates and solvates, in the form of a solid dispersion. The matrix of the solid dispersion is a polymer selected from at least one of polyvinylpyrrolidone, copovidone, hydroxypropyl cellulose, hydroxypropyl methylcellulose, polyethylene glycol, and polyethylene oxide.

CN105126111A discloses a sorafenib pharmaceutical composition at molecular level, which comprises an active ingredient, wherein the active ingredient is selected from at least one of sorafenib, sorafenib salt, sorafenib derivative or prodrug thereof; and a polymer cosolvent, wherein the polymer cosolvent has vinyl acetate group, such as VA64, polyvinylcaprolactam-polyvinyl acetate-polyethylene glycol graft copolymer.

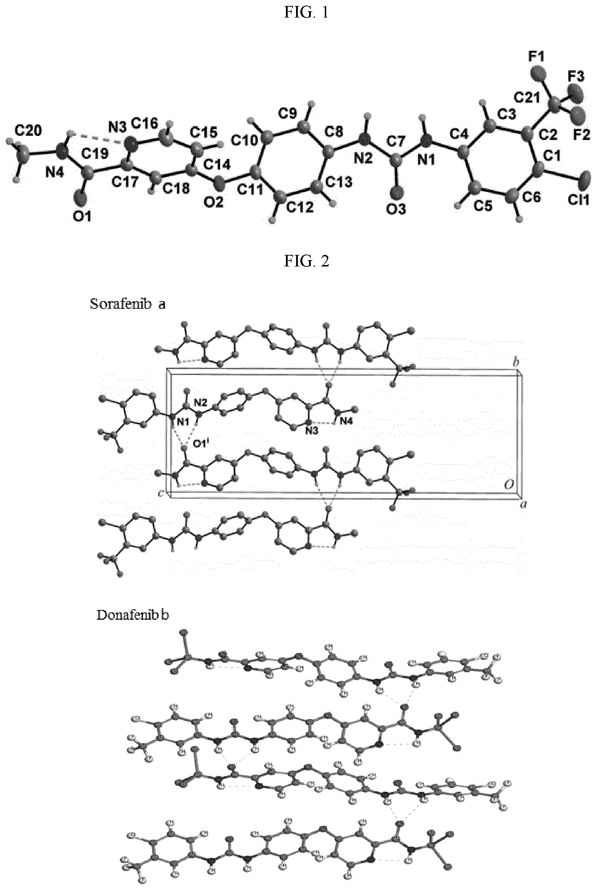

Compared with Nexavar Sorafenib Tosylate Tablets, the in vitro dissolution of the pharmaceutical composition of sorafenib or donefenib at molecular level in the prior art is improved to some extent, but there are still the following problems:
1) The increase in bioavailability in the human body is not as significant as that in the in vitro dissolution. For example, in WO2018211336A2, in healthy volunteers, the oral bioavailability is approximately doubled compared with the reference preparation Nexavar Sorafenib Tosylate Tablets (Cmax, AUC₇₂ₕ, T/R are 194.32% and 199.58% respectively). The patent did not disclose the number of subjects and the administration dosage of self-made products and reference preparation. According to the instructions of Nexavar Sorafenib Tosylate Tablets or Zepsun Donafenib Tosylate Tablets, the technology solved part of the solubility problem.
2) There is no data on significantly improved bioavailability in humans. The bioavailability data are available only in beagle dogs or rats. For example, in the Examples of CN106667916B, the AUC in rats is 2.24 times that of oral suspension; in CN105126111A, its optimal formulation improved Cₘₐₓ and AUC distribution by 85% and 78% over simulated commercially available preparations in beagles. It is well known that although bioavailability is improved in rats or beagles, it may not be improved in humans either, and numerous tests have confirmed that there is no necessary correlation. To confirm whether it is feasible in humans, a lot of tests and money are required. In addition, the above-disclosed technology, even in rats and beagles, can only be improved by about twice, which is still insignificant compared to the safety requirements of clinical drug use.
3) There are only in vitro dissolution data, but no in vivo data. For example, CN107115317, CN104888228A, WO2006026501A1 only provide in vitro dissolution data of sorafenib. Pharmaceutical professionals all know that for in vitro dissolution experiments, especially for poorly soluble drugs, the final determined dissolution behavior and dissolution results will be very different due to the different volume of dissolution medium and the concentration of surfactant added in the medium to improve dissolution rate. Thus, the improved in vitro dissolution does not prove that bioavailability can be significantly improved in the human body.
4) The commercial specification of Sorafenib Tosylate Tablets is 200 mg, and the recommended dosage is 400 mg each time. The specifications are large, and the administration dosage is high. When preparing a solid dispersion, it is necessary to add a carrier with a mass ratio several times that of the amount of active ingredients. For example, in the patent WO2006026501A1, the dissolution rate can only reach 35% when the polymer material povidone K25 is used at an amount five times the amount of active ingredient. As a result, even in the formulation of the dosage form prepared according to the target specification of 100 mg, the amount of the solid dispersion composition reaches 600 mg, and most of the commonly used solid dispersion carriers have a binding effect or form gels, which leads to the difficulty of preparation molding. For example, the disintegration time of tablets and capsules is greatly prolonged. In order to improve disintegration, a large number of adjuvants of the preparation need to be added, resulting in the large volume and weight of the tablets or the content of capsules. Therefore, the preparation is unfavorable for patients to take, resulting in poor patient compliance and high cost of the improved product. However, reducing the amount of carrier in the solid dispersion, on the one hand, will not have a significant solubilization effect and will also reduce the physical stability of the product, as shown in Example 2 of the patent WO2006026501A1, and on the other hand, will lead to incomplete formation of the solid dispersion (crystalline drugs fail to be completely converted to amorphous forms) from the perspective of preparation technology. In this case, once the crystal nuclei of sorafenib crystalline form exist, the drug will rapidly crystallize in a short period of time in the subsequent storage process or the physical stability will decrease, thus affecting the curative effect of the drug, and even failing to be developed and marketed as a real product. Therefore, it is not an easy task that how to break through the limitations of the prior art and how to develop a new generation of sorafenib therapeutic drug with good stability, bioavailability and patient compliance using the least amount of carriers or adjuvants in view of the objective fact of high clinical dosage of sorafenib tosylate tablets and large amount of adjuvants per unit of preparation. This is also a serious limitation in the prior art.
5) Solid dispersions, solid solutions and nanocrystal preparations are sensitive to moisture, and high moisture content accelerates drug nucleation, crystallization and chemical degradation, resulting in poor stability of the preparation. In addition, sorafenib itself has poor stability, and it is susceptible to degradation to generate mutagenic impurity CTF-aniline, which is more likely to be generated after sorafenib is prepared into a solid dispersion. The structure of CTF-aniline is shown in Formula (3): 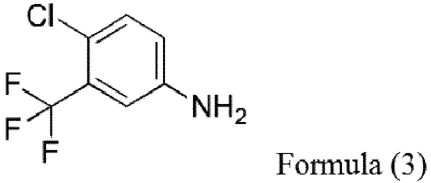
6) For the sorafenib solid dispersion pharmaceutical composition prepared using polymer carrier, the solid dispersion can be rapidly dissolved in vitro or in vivo. However, due to the intermolecular interaction of sorafenib and the intermolecular interaction between sorafenib and toluene sulfonic acid, molecular packing occurs rapidly in the solution through intermolecular noncovalent interactions such as hydrogen bonds and ionic interactions to form a supramolecular self-assembly system which is rapidly precipitated from the solution or intestinal fluid, ultimately resulting in a very low concentration of sorafenib molecules in the solution (pH 1.0: 0.0034 mg/ml, pH 4.5: 0.00013 mg/ml). In a dissolution experiment using pH 6.8 phosphate buffer containing 0.1% sodium dodecyl sulfate as surfactant (the amount of surfactant used is at least 10 times lower than that in the quality control dissolution medium of sorafenib tosylate), at 37°C and 2-4 h, the accumulative dissolution rate decreased to basically the same level as that of Nexavar Sorafenib Tablets, or even lower (that is, the spring effect often referred to in the prior art), resulting in an insignificant improvement of the in vivo bioavailability of the solid dispersion pharmaceutical composition.
   Acta Cryst. (2011). C67, o29-o32, ACS Omega. 2021 Mar 2; 6(8): 5532-5547 reported the structure of sorafenib tosylate single crystal and the supramolecular self-assembled system constructed by the intermolecular synergistic regulation of diaryl urea and dimethyl sulfoxide in the molecular structure of sorafenib. The chemical structure of sorafenib contains diaryl urea, and the planarization of the two aryl planes in the diaryl urea contributes to the formation of strong π-π stacking, hydrophobic effect and hydrogen bonding between sorafenib molecules, making sorafenib molecules easily undergo rapid molecular packing in the solution through intermolecular interaction and form a stable solid supramolecular self-assembled system, as shown in FIG. 2. From the perspective of drug developers, the supramolecular self-assembled system formed by π-π stacking is the most difficult to break. Although the existing technical means such as dispersion technology, cocrystal, salt forming and other technologies can instantly break the supramolecular self-assembled structure formed by π-π stacking, they cannot maintain the stability on the time axis required for practical application. AAPS Pharm Sci Tech (2022) 23:16 summarizes the existing technologies to solve the solubility of insoluble drugs, but none of these technologies can solve the pain points in the clinical application of currently marketed products of sorafenib. ACS Appl. Mater. Interfaces 2019, 11, 43996-44006 and Molecular Pharmaceutics, 2015, 12 (3): 922-931, Scientifc Reports | (2021) 11:874 respectively reported using self-assembled systems constructed from sorafenib/curcumin/PEG-VES(PEG-Vitamin E copolymer), sorafenib/Indocyanine, sorafenib and peptides to improve the efficacy and bioavailability of sorafenib, but these studies are still limited to academic research, and there is still a long way to go for product transformation.
7) Using a solid dispersion together with cosolvents or carriers with inhibiting CYP3A4 or P-gp inhibitory effects, such as tocopherol compounds, polyol fatty acid esters, polyalkoxylated fatty alcohol ethers, hydrogenated castor oil and other cosolvents or enzyme inhibitors. Due to the low melting point of these cosolvents, the heat generated during the preparation production increases the difficulty of the production process, and additionally, the physical stability of the solid dispersion of sorafenib will decrease. The amorphous sorafenib with poor thermodynamic stability during long-term storage will easily be induced by cosolvents to accelerate nucleation and crystallization and precipitate in large quantities, which makes it impossible to significantly improve the bioavailability of the drug and reduce the dosage. In addition, although hydrogenated castor oil and tocopherol have inhibitory effects on CYP3A4 or P-gp, their specificity is not strong, and they need to be added in a large amount to have a certain effect.
8) Chinese patents CN103301066 B and CN103301067 B disclosed the patents of donafenib preparation composition of Jiangsu Zelgen Pharmaceuticals Co., Ltd. It can be seen that the donafenib marketed product uses solid dispersion technology to improve its solubility and double the oral bioavailability of sorafenib. Therefore, the clinical dosage is reduced by half on the basis of Nexavar Sorafenib Tosylate Tablets. Since deuterated intermediates are used in the preparation process of active pharmaceutical ingredients of donafenib, and the price of deuterated intermediates is significantly higher than that of non-deuterated intermediates, it is of great significance to improve the oral absorption efficiency of donafenib preparation products and reduce the amount of active pharmaceutical ingredient (API) per unit preparation cost and reduce the adverse reactions of donafenib preparation products. According to this patent, the preparation of the solid dispersion of donafenib tosylate requires at least a ratio of main drug to high molecular polymer of 2-4: 1, meaning that when the amount of the main drug in a unit preparation is 274 mg, the amount of high molecular polymer used to prepare the solid dispersion is at least 548 mg-1096 mg. Otherwise it is difficult to solve the solubility and dissolution of the drug and physicochemical stability of the drug within shelf life.

Besides, Nexavar currently marketed in China is administered twice a day on an empty stomach or with a low-fat or medium-fat diet. According to the instruction of Nexavar in the United States, it is administered twice a day without food (at least 1 h before meal or 2 h after meal). Regarding administration with low-fat diets and medium-fat diets, there are difficulties in judging in the actual administration. Even professionals often have difficulty in judging the difference between a low-fat, medium-fat diet and a high-fat diet, let alone patient. This results in patients often taking the drug on an empty stomach, such as taking the drug at least 1 h before meal or 2 h after meal as required by the instructions of Nexavar in the United States. However, when taken on an empty stomach, the higher local concentration of the drug will result in a higher incidence of toxic and side effects in the upper part of the digestive tract, greater severity of side effects, and a higher incidence of grade 3 and 4 serious adverse reactions (e.g., Seminars in Oncology, Vol 41, No S2, February 2014, pp S1-S16). As mentioned above (Oncotarget, 2017, Vol. 8, (26), pp: 43458-43469), the incidence of diarrhea after taking sorafenib was up to 76.5%. As described in the literature (Hua Su, Xiaodong Zhou, Aihua Ma, et al. Gastrointestinal adverse reactions caused by commonly used drugs and their prevention [J]. Adverse Drug Reactions Journal, 2004, 6(1):5.), taking drugs on an empty stomach is one of the main causes of gastrointestinal adverse reactions, while taking drugs with meals can reduce the irritation of drugs to gastrointestinal tract and reduce side effects. For example, "How to read drug instructions No. 12: Be knowledgeable about taking drugs with meals" (https://www.nmpa.gov.cn/xxgk/kpzhsh/kpzhshyp/20160503170501830.html) published by NMPA shows that taking drugs with meals can reduce the occurrence of adverse reactions of drugs, because taking them with food or immediately after meals can greatly reduce the irritation of drugs to the gastrointestinal tract. Therefore, the development of sorafenib that can be taken with meals is of great significance to reduce the adverse reactions and reduce the dose reduction of the drug caused by gastrointestinal adverse reactions.

Moreover, according to Variability of Sorafenib Toxicity and Exposure over Time: A Pharmacokinetic /Pharmacodynamic Analysis The Oncologist 2012;17:1204-1212, the current clinical therapeutic dose of Sorafenib Tosylate Tablets is a fixed dose of 400 mg each time, twice a day (800 mg per day). However, with the extension of treatment time, the drug exposure of existing marketed products gradually decreases. For example, taking drug for 120 days resulted in a decrease in drug exposure of about 38% compared to taking drug for 15 days. The drug exposure is directly related to the clinical treatment effect (overall survival period, progression-free survival, time to treatment failure). When disease progression occurs during clinical treatment of existing marketed products, the clinical dosage will be increased continuously. For example, when the dosage increased from 400 mg each time to 600 mg each time, the drug exposure of sorafenib only increased by 13% limited by drug solubility and dissolution, which has no clinical significance (FDA Review Report Bio Pharmacology Review), but grade 3 or 4 serious adverse reactions increased significantly, including severe gastrointestinal toxicity and skin toxicity, indicating that when the disease progresses with clinical treatment using existing marketed products, it is impossible to carry out dose titration on the basis of the current treatment regimen to maintain the optimal therapeutic effect. Therefore, overcoming the drug resistance of sorafenib/donafenib and facilitating dose titration are of great significance in maximizing the efficacy of the product, which is an urgent clinic problem to be solved.

Therefore, it is urgent to develop a new sorafenib preparation that can solve the clinical drug resistance of sorafenib or donafenib, make its absorption not affected by food or gastrointestinal enzymes (CYP3A4 or P-gp, etc.), and have stable efficacy, which will have significant clinical advantages over existing marketed preparations.

### SUMMARY

The technical problem solved by the present disclosure is to provide an oral preparation of sorafenib or donafenib. The oral preparation of sorafenib or donafenib provided by the present application can be administered with a low dose, and it has good physical and chemical stability, low Cₘₐₓ and AUC₀₋ₜ variation, and the absorption is not affected by food. When a patient's disease progresses, dose titration can be used to maintain the best efficacy and overcome the durability problem, thereby significantly extending the patient's progression-free survival, improving the patient's overall survival, and delaying time to treatment failure with more significant efficacy and safety. In addition, the patient compliance is also improved.

In view of this, the present application provides an oral preparation of sorafenib or donafenib with low administration dosage, comprising a pharmaceutical composition of sorafenib or donafenib at molecular level, a self-assembled polymer material and an additional pharmaceutically acceptable adjuvant, wherein the pharmaceutical composition of sorafenib or donafenib at molecular level is a solid dispersant, solid solution, polymer nanoparticle, self-microemulsion, nanoemulsion at molecular level or a mixture at molecular level;
wherein, the self-assembled polymer material is selected from the group consisting of povidone + hydroxypropyl cellulose, povidone with low viscosity + povidone with high viscosity, povidone + copovidone, povidone + polyvinylcaprolactam-polyvinyl acetate-polyethylene glycol graft copolymer, povidone + hydroxypropyl methylcellulose acetate succinate, povidone + hydroxypropyl methylcellulose, povidone + cellulose acetate, povidone + croscarmellose sodium, povidone + polyethylene glycol, povidone + low-substituted hydroxypropyl cellulose, povidone + hydroxypropyl methylcellulose phthalate, povidone + methacrylic acid-ethylacrylate copolymer, povidone + methacrylic acid-methacrylate copolymer, copovidone + hydroxypropyl cellulose, copovidone + polyvinylcaprolactam-polyvinyl acetate-polyethylene glycol graft copolymer, copovidone + hydroxypropyl methylcellulose acetate succinate, copovidone + hydroxypropyl methylcellulose, copovidone + cellulose acetate, copovidone + croscarmellose sodium or croscarmellose calcium, copovidone + polyethylene glycol, copovidone + low-substituted hydroxypropyl cellulose, copovidone + hydroxypropyl methylcellulose phthalate, copovidone + methacrylic acid-ethylacrylate copolymer, copovidone + methacrylic acid-methacrylate copolymer, polyvinylcaprolactam-polyvinyl acetate-polyethylene glycol graft copolymer + hydroxypropyl methylcellulose acetate succinate, polyvinylcaprolactam-polyvinyl acetate-polyethylene glycol graft copolymer + hydroxypropyl methylcellulose, polyvinylcaprolactam-polyvinyl acetate-polyethylene glycol graft copolymer + hydroxypropyl cellulose, polyvinylcaprolactam-polyvinyl acetate-polyethylene glycol graft copolymer + low-substituted hydroxypropyl cellulose, polyvinylcaprolactam-polyvinyl acetate-polyethylene glycol graft copolymer + croscarmellose sodium or croscarmellose calcium, polyvinylcaprolactam-polyvinyl acetate-polyethylene glycol graft copolymer + polyethylene glycol, polyvinylcaprolactam-polyvinyl acetate-polyethylene glycol graft copolymer + hydroxypropyl methylcellulose phthalate, polyvinylcaprolactam-polyvinyl acetate-polyethylene glycol graft copolymer + methacrylic acid-ethylacrylate copolymer, and polyvinylcaprolactam-polyvinyl acetate-polyethylene glycol graft copolymer + methacrylic acid-methacrylate copolymer.

Preferably, the self-assembled polymer material accounts for 10-50wt% of the pharmaceutical composition of sorafenib or donafenib at the molecular level.

Preferably, the hydroxypropyl methylcellulose is HPMC E5 or HPMCK100LV; the self-assembled polymer material is a self-assembled polymer material compounded with one of povidone, copovidone, polyvinylcaprolactam-polyvinyl acetate-polyethylene glycol graft copolymer and hydroxypropyl methylcellulose acetate succinate H type with strong hydrophobicity, and HPMC E5, at a compounding ratio of (1-10): 1; or the self-assembled polymer material is a self-assembled polymer material compounded with one of polyvinylcaprolactam-polyvinyl acetate-polyethylene glycol graft copolymer and hydroxypropyl methylcellulose acetate succinate H type with strong hydrophobicity, and HPMCK100LV, at a compounding ratio of (1-10): 1.

Preferably, the hydroxypropyl methylcellulose acetate succinate has an acetyl substitution amount of 6-16% and a succinyl substitution amount of 4-16%.

Preferably, the povidone has an average molecular weight of 34,000 to 1,300,000, or the povidone is compounded with povidone with an average molecular weight of 34,000 and povidone with an average molecular weight of 1,300,000 at a ratio of 1:1-1:0.1.

Preferably, a unit dose of the oral preparation contains 20-69 mg of sorafenib or donafenib.

Preferably, the pharmaceutical composition of sorafenib or donafenib at molecular level comprises sorafenib or donafenib, or a salt, hydrate, or solvate thereof, or a hydrates or solvate of a salt thereof, and a carrier; the carrier is selected from the group consisting of copovidone, povidone, polyvinylcaprolactam-polyvinyl acetate-polyethylene glycol graft copolymer, povidone + hydroxypropyl methylcellulose acetate succinate, povidone + hydroxypropyl methylcellulose, povidone with low viscosity + povidone with high viscosity, povidone + copovidone, povidone + polyvinylcaprolactam-polyvinyl acetate-polyethylene glycol graft copolymer, copovidone + hydroxypropyl methylcellulose acetate succinate, copovidone + polyvinylcaprolactam-polyvinyl acetate-polyethylene glycol graft copolymer, copovidone + hydroxypropyl methylcellulose, copovidone + polyethylene glycol, copovidone + hydroxypropyl cellulose, copovidone + cellulose acetate, polyvinylcaprolactam-polyvinyl acetate-polyethylene glycol graft copolymer + hydroxypropyl methylcellulose, polyvinylcaprolactam-polyvinyl acetate-polyethylene glycol graft copolymer + hydroxypropyl methylcellulose acetate succinate, polyvinylcaprolactam-polyvinyl acetate-polyethylene glycol graft copolymer + povidone, and polyvinylcaprolactam-polyvinyl acetate-polyethylene glycol graft copolymer + copovidone.

Preferably, the sorafenib or donafenib, or a salt, hydrate, or solvate thereof, or a hydrates or solvate of a salt thereof accounts for 30-60wt% of the pharmaceutical composition of sorafenib or donefenib at molecular level.

Preferably, a dosage form of the oral preparation of sorafenib or donafenib is tablet, suspension, granule or capsule.

Preferably, the tablet comprises an adjuvant selected from the group consisting of a filler, disintegrant, glidant and lubricant; the filler is selected from the group consisting of microcrystalline cellulose, silicified microcrystalline cellulose, and a mixture thereof; the disintegrant is croscarmellose sodium; the glidant is silicon dioxide; and the lubricant is selected from the group consisting of magnesium stearate, sodium stearyl fumarate, and a mixture thereof.

Preferably, a content of the filler is 30-45wt% of the tablet, a content of the disintegrant is 5-15wt% of the tablet, a content of the glidant is 3-5wt% of the tablet, and a content of the lubricant is 1-5wt% of the tablet.

The present application also provides use of the oral preparation of sorafenib or donafenib in the manufacture of a medicament for treating or alleviating hepatocellular carcinoma, breast cancer, primary malignant liver tumor, cholangiocarcinoma, renal cell carcinoma, recurrent or refractory solid tumor, central nervous solid tumor, metastatic non-small cell lung cancer, unresectable hepatocellular carcinoma, thyroid cancer, pancreatic cancer, adenoid cystic carcinoma of the salivary gland, non-differentiated thyroid carcinoma, refractory differentiated thyroid cancer, adenoid cystic carcinoma, advanced endometrial cancer, anaplastic thyroid cancer, rectal cancer, colon cancer, unresectable stage III or IV melanoma, leukemia, acute myeloid leukemia, all solid tumors or advanced solid tumors.

The present application provides an oral preparation of sorafenib or donafenib, which comprises a pharmaceutical composition of sorafenib or donafenib at molecular level, a self-assembled polymer material and an additional pharmaceutically acceptable adjuvant, wherein the self-assembled polymer material uses a compound of two specific polymer materials as the compounding self-assembled construction unit. The two polymer materials are coordinated with the sorafenib molecule to reconstruct a reversible self-assembled system based on weak intermolecular interactions, which improves the solubility and stability of sorafenib or donafenib, and also reduces the premature metabolism and efflux of drugs by CYP3A4/5, CYP2C8/D6 and P-gp, so that the obtained new oral preparation of sorafenib or donafenib can be taken at a lower dose to achieve higher drug exposure, and has the characteristics of safety, less incidence of side effects, low Cₘₐₓ and AUC₀₋ₜ variation, low weight/volume, rapid disintegration, good physical and chemical stability that it can remain stable even if the moisture content is up to 5%. In addition, its administration is not affected by food, and when a patient's disease progresses as the treatment time increases, dose titration can be used to achieve the best efficacy and overcome the drug resistance problem of the existing marketed products of sorafenib or donefenib during treatment.

### BRIEF DESCRIPTION OF DRAWINGS

FIG. 1 shows the three-dimensional structure of sorafenib of the present disclosure.
FIG. 2 shows the single-crystal structure of the intermolecular interaction between sorafenib and donafenib of the present disclosure.
FIG. 3 is a graph showing the regulation of SLFN molecular packing by the single polymer self-assembled construction unit at high concentrations in an embodiment of the present disclosure.
FIG. 4 is a graph showing the synergistic regulation of sorafenib molecular packing by the copovidone-compounded polymer self-assembled construction unit in an embodiment of the present disclosure.
FIG. 5 is a graph showing the synergistic regulation of sorafenib molecular packing by the low-viscosity povidone-compounded polymer self-assembled construction unit in an embodiment of the present disclosure.
FIG. 6 is a graph showing the synergistic regulation of sorafenib molecular packing by the HPMC AS-compounded polymer self-assembled construction unit in an embodiment of the present disclosure.
FIG. 7 is a graph showing the synergistic regulation of sorafenib molecular packing by the HPMC-compounded polymer self-assembled construction unit in an embodiment of the present disclosure.
FIG. 8 is a graph showing the synergistic regulation of sorafenib molecular packing by the PEG-compounded polymer self-assembled construction unit in an embodiment of the present disclosure.
FIG. 9 is a graph showing the synergistic regulation of sorafenib molecular packing by the HPMCAS-H-compounded polymer self-assembled construction unit in an embodiment of the present disclosure.
FIG. 10 is a graph showing the synergistic regulation of sorafenib molecular packing by the HPMCAS-L-compounded polymer self-assembled construction unit in an embodiment of the present disclosure.
FIG. 11 is a graph showing the synergistic regulation of sorafenib molecular packing by the HPMCE5-compounded polymer self-assembled construction unit in an embodiment of the present disclosure.
FIG. 12 is a bar graph showing the effects of the self-assembled construction units compounded with HPMC E5 and other polymers at different ratios on synergistic regulation and stability of sorafenib molecular packing for 6 h in an embodiment of the present disclosure.
FIG. 13 is a drug-time curve showing different preparations and the reference preparation in healthy subjects in an embodiment of the present disclosure.
FIG. 14 shows the comparison of PK curves of preparation T1 of the present disclosure and reference preparation Nexavar in healthy subjects on an empty stomach and a high-fat diet.

### DETAILED DESCRIPTION

In order to further understand the present disclosure, preferred embodiments of the present disclosure will be described in combination with examples below, but it should be understood that these descriptions are only for further illustrating the features and advantages of the present disclosure, and are not intended to limit the claims of the present disclosure.

In view of the problems existing in the solid preparations of sorafenib or donefenib in the prior art, the present application provides innovative solutions and ideas for addressing the problems of large-scale insolubility, high variation in vivo, molecular packing of drug molecules themselves, high variation in drug absorption or low bioavailability of oral drugs caused by intestinal P450 enzymes/P-gp, thereby maximizing the effectiveness and safety of the drug. Therefore, the present application specifically provides an oral preparation of sorafenib or donafenib, which by using the compounding self-assembled construction unit and combining a molecular-level pharmaceutical composition technology, allows the obtained oral preparation of sorafenib or donafenib can be administered at a low dose, that is to say, the same curative effect as the ideal curative effect can be achieved with only 10%-35% of the administered dose of Nexavar Sorafenib Tosylate Tablets or a higher drug exposure can be obtained with 40%-60% of the administered dose of the existing donafenib tablets. It has the characteristics of safety, less incidence of side effects, low Cₘₐₓ and AUC₀₋ₜ variation, low weight/volume, rapid disintegration, good physical and chemical stability that it can remain stable even if the moisture content is up to 5%. In addition, its administration is not affected by food, and when a patient's disease progresses as the treatment time increases, dose titration can be used to achieve the best efficacy and overcome the drug resistance problem of the existing marketed products of sorafenib or donefenib during treatment.

Specifically, an embodiment of the present disclosure discloses an oral preparation of sorafenib or donafenib with low administration dosage, comprising a pharmaceutical composition of sorafenib or donafenib at molecular level, a self-assembled polymer material and an additional pharmaceutically acceptable adjuvant, wherein the pharmaceutical composition of sorafenib or donafenib at molecular level is a solid dispersant, solid solution, polymer nanoparticle, self-microemulsion, nanoemulsion at molecular level or a mixture at molecular level.

The self-assembled polymer material described in the present application, also known as the compounding self-assembled construction unit, is selected from the group consisting of povidone + hydroxypropyl cellulose, povidone with low viscosity + povidone with high viscosity, povidone + copovidone, povidone + polyvinylcaprolactam-polyvinyl acetate-polyethylene glycol graft copolymer, povidone + hydroxypropyl methylcellulose acetate succinate, povidone + hydroxypropyl methylcellulose, povidone + cellulose acetate, povidone + croscarmellose sodium, povidone + polyethylene glycol, povidone + low-substituted hydroxypropyl cellulose, povidone + hydroxypropyl methylcellulose phthalate, povidone + methacrylic acid-ethylacrylate copolymer, povidone + methacrylic acid-methacrylate copolymer, copovidone + hydroxypropyl cellulose, copovidone + polyvinylcaprolactam-polyvinyl acetate-polyethylene glycol graft copolymer, copovidone + hydroxypropyl methylcellulose acetate succinate, copovidone + hydroxypropyl methylcellulose, copovidone + cellulose acetate, copovidone + croscarmellose sodium or croscarmellose calcium, copovidone + polyethylene glycol, copovidone + low-substituted hydroxypropyl cellulose, copovidone + hydroxypropyl methylcellulose phthalate, copovidone + methacrylic acid-ethylacrylate copolymer, copovidone + methacrylic acid-methacrylate copolymer, polyvinylcaprolactam-polyvinyl acetate-polyethylene glycol graft copolymer + hydroxypropyl methylcellulose acetate succinate, polyvinylcaprolactam-polyvinyl acetate-polyethylene glycol graft copolymer + hydroxypropyl methylcellulose, polyvinylcaprolactam-polyvinyl acetate-polyethylene glycol graft copolymer + hydroxypropyl cellulose, polyvinylcaprolactam-polyvinyl acetate-polyethylene glycol graft copolymer + low-substituted hydroxypropyl cellulose, polyvinylcaprolactam-polyvinyl acetate-polyethylene glycol graft copolymer + croscarmellose sodium or croscarmellose calcium, polyvinylcaprolactam-polyvinyl acetate-polyethylene glycol graft copolymer + polyethylene glycol, polyvinylcaprolactam-polyvinyl acetate-polyethylene glycol graft copolymer + hydroxypropyl methylcellulose phthalate, polyvinylcaprolactam-polyvinyl acetate-polyethylene glycol graft copolymer + methacrylic acid-ethylacrylate copolymer, and polyvinylcaprolactam-polyvinyl acetate-polyethylene glycol graft copolymer + methacrylic acid-methacrylate copolymer.

The present application provides an oral preparation of sorafenib or donefenib, wherein the compounding self-assembled construction unit accounts for 10-50wt% of the pharmaceutical composition of sorafenib at molecular level. More specifically, the compounding self-assembled construction unit accounts for 20-40wt% of the pharmaceutical composition of sorafenib or donafenib at molecular level.

The pharmaceutical composition of sorafenib or donefenib at molecular level exists in the form of a solid dispersant, solid solution, polymer nanoparticle, self-microemulsion, nanoemulsion at molecular level or a mixture at molecular level. The pharmaceutical composition of sorafenib or donafenib at molecular level comprises sorafenib or donafenib, or a salt, hydrate, or solvate thereof, or a hydrates or solvate of a salt thereof, and a carrier.

In the present application, the sorafenib or donafenib, or a salt, hydrate, or solvate thereof, or a hydrates or solvate of a salt thereof accounts for 30-60wt% of the pharmaceutical composition of sorafenib or donefenib at molecular level. More specifically, the sorafenib, or a salt, hydrate, or solvate thereof, or a hydrate or solvate of a salt thereof accounts for 35-55wt% of the pharmaceutical composition of sorafenib or donefenib at molecular level.

In the pharmaceutical composition of sorafenib or donafenib at molecular level, the carrier is selected from a polymer carrier which can provide hydrogen acceptor and has a certain proportion of hydrophobic groups, and/or a combination of at least two polymer carriers, specifically selected from the group consisting of copovidone, povidone, polyvinylcaprolactam-polyvinyl acetate-polyethylene glycol graft copolymer (Soluplus), povidone and hydroxypropyl methylcellulose acetate succinate (HPMCAS), povidone and hydroxypropyl methylcellulose, povidone with low viscosity and povidone with high viscosity, povidone and copovidone, povidone and polyvinylcaprolactam-polyvinyl acetate-polyethylene glycol graft copolymer, copovidone and hydroxypropyl methylcellulose acetate succinate, copovidone and polyvinylcaprolactam-polyvinyl acetate-polyethylene glycol graft copolymer, copovidone and hydroxypropyl methylcellulose (HPMC), copovidone and polyethylene glycol (PEG), copovidone and hydroxypropyl cellulose, copovidone and cellulose acetate, polyvinylcaprolactam-polyvinyl acetate-polyethylene glycol graft copolymer and hydroxypropyl methylcellulose, polyvinylcaprolactam-polyvinyl acetate-polyethylene glycol graft copolymer and hydroxypropyl methylcellulose acetate succinate, polyvinylcaprolactam-polyvinyl acetate-polyethylene glycol graft copolymer and povidone, and polyvinylcaprolactam-polyvinyl acetate-polyethylene glycol graft copolymer and copovidone. More specifically, the carrier is selected from the group consisting of PVP K30, HPC LF, HPMC E5, VA64, PVP K25+K90, PVP K25+VA64, PVP K30+HPMC AS, PVP K90+HPMC AS, Soluplus and VA64+HPMC AS. In a specific embodiment, the mass ratio of sorafenib or donafenib, or a salt, hydrate, or solvate thereof, or a hydrate or solvate of a salt thereof to the carrier is 1: 1.

In the present application, the hydroxypropyl methylcellulose in the compounding self-assembled construction unit is preferably E series or K series, wherein the E series is more preferably a low-viscosity model, and the K series is more preferably K100LV. The compounding self-assembled construction unit is a compounding self-assembled construction unit compounded with one of povidone, copovidone, polyvinylcaprolactam-polyvinyl acetate-polyethylene glycol graft copolymer and hydroxypropyl methylcellulose acetate succinate with strong hydrophobicity, and HPMC E5, at a compounding ratio of (1-10): 1; or the compounding self-assembled construction unit is a compounding self-assembled construction unit compounded with one of polyvinylcaprolactam-polyvinyl acetate-polyethylene glycol graft copolymer and hydroxypropyl methylcellulose acetate succinate with strong hydrophobicity, and HPMC K100LV, at a compounding ratio of (1-10): 1. The hydroxypropyl methylcellulose acetate succinate has an acetyl substitution amount of 6-16% and a succinyl substitution amount of 4-16%. More specifically, the hydroxypropyl methylcellulose acetate succinate has an acetyl substitution amount of 10-14% and a succinyl substitution amount of 4-8%. In the crystallization inhibitor, the povidone has an average molecular weight of 34,000 to 1,300,000, or the povidone is compounded with povidone with an average molecular weight of 34,000 and povidone with an average molecular weight of 1,300,000 at a ratio of 1: 1-1:0.1.

In a specific embodiment, the compounding self-assembled construction unit is specifically selected from the group consisting of PVP VA64 + HPMCAS H type, PVP VA64 + HPMCAS M type, PVP VA64 + Soluplus, PVP VA64 + HPMC, PVP K90 + HPMCAS, PVP K90 + HPMC, PVP K25 + PVP K90, PVP K30 + PVP K90, HPMC with low viscosity + PVP VA64, PVP K90 + HPMC K100LV, + povidone, HPMCAS H type + Soluplus, HPMCAS M type + HPMCAS L type +Soluplus, HPMC (low viscosity) + povidone, HPMC (low viscosity) + copovidone, and HPMC (low viscosity)+ HPMCAS (high hydrophobicity).

In the present application, the compounding self-assembled construction unit can be added internally or externally, or one internally and the other externally, and the compounding self-assembled construction unit can be added into the coating, such as powder coating, pellet coating or tablet coating.

In the sorafenib oral preparation described in the present application, the dosage of the sorafenib pharmaceutical composition at the molecular level is 10%-35%, preferably 12.5-30% of the administration dosage of Nexavar tablets. The unit dose of the oral preparation contains 20 mg-69 mg of sorafenib or donafenib, which is equivalent to 200 mg of Nexavar tablets, preferably 25 mg, 30 mg, 50 mg or 60 mg. The unit preparation specification is 50-140 mg, which is equivalent to a single dose of 400 mg of Nexavar tablets, preferably 50 mg, 60 mg, 100 mg or 120 mg.

In the donafenib oral preparation described in the present application, the dosage of the donafenib pharmaceutical composition at the molecular level is 40%-60% of the administration dosage of Zepsun Donafenib Tosylate Tablets, preferably 45%-55%. The unit preparation specification is preferably 25 mg, 30 mg, 40 mg, 45 mg, 50 mg or 55 mg, which is equivalent to a single dose of 100 mg of Zepsun Donafenib Tosylate Tablets, preferably 50 mg or 60 mg. which is equivalent to a single dose of 200 mg of Zepsun Donafenib Tosylate Tablets, preferably 80 mg, 90 mg, 100 mg, or 110 mg.

The dosage form of the oral preparation of sorafenib or donefenib is tablet, suspension, granule or capsule. The adjuvant of the tablet includes filler, disintegrant, glidant or lubricant. The filler is selected from the group consisting of microcrystalline cellulose and silicified microcrystalline cellulose, and a mixture thereof. The disintegrant is croscarmellose sodium. The glidant is silicon dioxide. The lubricant is selected from the group consisting of magnesium stearate, sodium stearyl fumarate, and a mixture thereof. The content of the filler is 30-45wt% of the tablet, preferably 30-40wt%. The content of the disintegrant is 5-15wt% of the tablet, preferably 5-10wt%. The content of the glidant is 3-5wt% of the tablet. The content of the lubricant is 1-5wt% of the tablet. In the present application, the disintegration time of the tablet is ≤ 5 min.

The present disclosure also provides use of the above described preparation containing the sorafenib composition in the manufacture of a medicament for treating tumors, wherein the tumor is specifically hepatocellular carcinoma, breast cancer, primary malignant liver tumor, cholangiocarcinoma, renal cell carcinoma, recurrent or refractory solid tumor, central nervous solid tumor, metastatic non-small cell lung cancer, unresectable hepatocellular carcinoma, thyroid cancer, pancreatic cancer, adenoid cystic carcinoma of the salivary gland, non-differentiated thyroid carcinoma, refractory differentiated thyroid cancer, adenoid cystic carcinoma, advanced endometrial cancer, anaplastic thyroid cancer, rectal cancer, colon cancer, unresectable stage III or IV melanoma, leukemia, acute myeloid leukemia, all solid tumors or advanced solid tumors.

The preparation provided by the present disclosure can be used alone or in combination with an additional therapeutic for treating the above-mentioned tumors; wherein the additional therapeutic is preferably PD-1 inhibitors, venetoclax and/or decitabine, combined therapy with TACE and/or Iodion-125 seeds brachytherapy, combined therapy with drug-eluting bead transarterial chemoembolization, combined therapy with pembrolizumab, combined therapy with toripalimab, combined therapy with atezolizumab, combined therapy with atezolizumab + bevacizumab, combined therapy with TACE + tilelizumab, combined therapy with recombinant human adenovirus type 5 injection, combined therapy with MTL-CEBPA, combined therapy with vemurafenib, combined therapy with oxaliplatin and 5-fluorouracil, eribulin, toripalimab, gemox or everolimus, combined therapy with doxorubicin and lipiodol, combined therapy with oxaliplatin, raltitrexed and epirubicin, combined therapy with toripalima, combined therapy with PD-1 mAb, combined therapy with hydroxychloroquine, combined therapy with CVM-1118, combined therapy with hepatic artery infusion chemotherapy (oxaliplatin, fluorouracil, and leucovorin), combined therapy with hepatic artery infusion chemotherapy (irinotecan, oxaliplatin, fluorouracil, and leucovorin), combined therapy with YIV-906, or combined therapy with tislelizumab.

The clinical application of sorafenib or donafenib as single agent or combination therapy in various disease treatment fields can be further expanded.

For example, the oral preparation of sorafenib or donafenib with low administration dosage of the present disclosure can be used as a first-line treatment drug for advanced liver cancer in combination with a PD-1 inhibitor such as camrelizumab. Camrelizumab is intravenously injected at 200 mg on the first day, once every two weeks, and the pharmaceutical composition of sorafenib or donafenib is administered orally at 50 mg-120 mg the next day, twice a day.

The oral preparation of sorafenib or donafenib with low administration dosage of the present disclosure is used for the treatment of hepatocellular carcinoma in combination with TACE therapy. TACE therapy is carried out to inject chemotherapy drugs (doxorubicin and lipiodol), and sorafenib or donafenib is administered orally at 50-120 mg each time, twice a day.

The oral preparation of sorafenib or donafenib with low administration dosage of the present disclosure is used for the treatment of unresectable hepatocellular carcinoma and portal vein tumor thrombus in combination with toripalimab. According to patients in cohort A or cohort B, sorafenib or donafenib is administered at 50-120 mg each time, once or twice a day.

The oral preparation of sorafenib or donafenib with low administration dosage of the present disclosure is used for the treatment of unresectable hepatocellular carcinoma and portal vein tumor thrombus in combination with SBRT+TACE in sequence. Sorafenib or donafenib is administered orally at 100-120 mg each time, twice a day.

The oral preparation of sorafenib or donafenib with low administration dosage of the present disclosure is used for the treatment of hepatocellular carcinoma in combination with hydroxychloroquine (HCQ). Sorafenib or donafenib is administered twice a day, 100-120 mg each time, and hydroxychloroquine (HCQ) is administered orally at 400 mg, once a day.

The oral preparation of sorafenib or donafenib with low administration dosage of the present disclosure is used for the treatment of advanced hepatocellular carcinoma in adults in combination with pembrolizumab. On the first day of treatment, pembrolizumab is intravenously injected within 30 min, and from the first to the 21st day of treatment, sorafenib or donafenib is administered orally at 100-120 mg each time, twice a day, and a course of treatment is repeated every three weeks.

The oral preparation of sorafenib or donafenib with low administration dosage of the present disclosure is used for the treatment of advanced hepatocellular carcinoma in combination with CVM-1118. CVM-1118 is administered orally at 150 mg or 200 mg, twice a day, and sorafenib or donafenib is administered orally at 50-120 mg each time, twice a day, with 28 days being a course of treatment.

The oral preparation of sorafenib or donafenib with low administration dosage of the present disclosure is used for the treatment of hepatocellular carcinoma in combination with hepatic arterial infusion chemotherapy (oxaliplatin, fluorouracil, and leucovorin). Sorafenib or donefenib is administered orally at 50-120 mg each time, twice a day.

The oral preparation of sorafenib or donafenib with low administration dosage of the present disclosure is used for the treatment of hepatocellular carcinoma in combination with hepatic arterial infusion chemotherapy (irinotecan, oxaliplatin, fluorouracil, and leucovorin). Sorafenib or donefenib is administered orally at 50-120 mg each time, twice a day.

The oral preparation of sorafenib or donafenib with low administration dosage of the present disclosure is used for the treatment of hepatocellular carcinoma in combination with DEB-TACE. Sorafenib or donefenib is administered orally at 50-120 mg each time, twice a day.

The oral preparation of sorafenib or donafenib with low administration dosage of the present disclosure is used to treat patients with advanced hepatocellular carcinoma infected with HBV in combination with YIV-906, with 28 days being a treatment cycle. Sorafenib or donefenib is administered orally at 50-120 mg each time, twice a day for 28 consecutive days, and YIV-906 is administered 4 days a week, twice a day, 600 mg each time, and stopped for 3 days.

The oral preparation of sorafenib or donafenib with low administration dosage of the present disclosure is used for the treatment of inoperable hepatocellular carcinoma in combination with TACE and tislelizumab. On the third to seventh day after the first TACE treatment, sorafenib or donafenib is administered orally at 100-120 mg each time, twice a day, and tislelizumab is also injected intravenously once every three weeks, 200 mg each time. The number of repeats of TACE treatment is determined based on clinical results, and treatment with tislelizumab is continued until 24 months.

The oral preparation of sorafenib or donafenib with low administration dosage of the present disclosure is used for the treatment of pancreatic cancer in combination with vemurafenib. Vemurafenib is administered orally at 720 mg, twice a day, and sorafenib or donafenib is administered at 100-120 mg every morning and sorafenib is administered at 50-60 mg every afternoon.

The oral preparation of sorafenib or donafenib with low administration dosage of the present disclosure is used for the treatment of hepatocellular carcinoma caused by virus infection in combination with MTL-CEBPA. MTL-CEBPA is injected intravenously at 130 mg/m² every week for three consecutive weeks with one week off, and sorafenib or donafenib is administered orally at 100-120 mg each time, twice a day.

The oral preparation of sorafenib or donafenib with low administration dosage of the present disclosure is used for the treatment of BCLC Stage C hepatocellular carcinoma in combination with TACE and tilelizumab. TACE treatment is started first. Tilelizumab is injected intravenously at 240 mg within one week of TACE treatment, once every three weeks, and sorafenib or donafenib is administered orally at 100-120 mg each time within one week of TACE treatment, twice a day.

The oral preparation of sorafenib or donafenib with low administration dosage of the present disclosure is used for the treatment of inoperable advanced hepatocellular carcinoma in combination with atezolizumab. Atezolizumab is intravenous injected at 1200 mg on the first day and the 21st day of the treatment cycle, and sorafenib or donafenib is administered orally every day at 100-120 mg twice a day during the treatment cycle. If effective, the treatment can be repeatedly for multiple courses until the disease progresses or is intolerable.

The oral preparation of sorafenib or donafenib with low administration dosage of the present disclosure is used for the treatment of hepatocellular carcinoma in combination with TACE. TACE therapy is carried out to inject chemotherapy drugs (200 mg of oxaliplatin, 4 mg of raltitrexed, 20 mg of epirubicin in cTACE or 70 mg in dTACE), and sorafenib or donafenib is administered orally at 50-120 mg each time, twice a day.

The oral preparation of sorafenib or donafenib with low administration dosage of the present disclosure is used for the treatment of inoperable hepatocellular carcinoma in combination with TACE therapy. On the third to seventh day after the first TACE treatment, sorafenib or donafenib is administered orally at 100-120 mg each time, twice a day. The number of repeats of TACE and sorafenib treatment is determined based on clinical results, and treatment with tislelizumab is continued until 24 months.

The oral preparation of sorafenib or donafenib with low administration dosage of the present disclosure is used for the treatment of acute myeloid leukemia (with a high FLT3-ITD allelic ratio) in adults in combination with venetoclax and decitabine. On the 1st-5th day of each treatment cycle, decitabine is injected intravenously at 20 mg/m². Meanwhile, venetoclax is administered orally at 100 mg, and the dose is gradually increased to 400 mg (day 28). This is one treatment cycle. If the patient has a high FLT3-ITD allelic ratio, on the third day of each treatment cycle, sorafenib or donafenib is administered at 100-120 mg each time, twice a day, until the end of a treatment cycle of 28 days.

The oral preparation of sorafenib or donafenib with low administration dosage of the present disclosure is used for the treatment of attacks and relapses of acute leukemia and hematopoietic stem cell transplantation. Sorafenib or donafenib is administered orally at 100-120 mg each time, twice a day, and the dose can be reduced to 50 mg-240 mg per day if not tolerable.

The oral preparation of sorafenib or donafenib with low administration dosage of the present disclosure is used for the treatment of advanced inoperable hepatocellular carcinoma in combination with HAIC (130 mg/m² of oxaliplatin and 2400 mg/m² of 5-fu, or 85 mg/m² of oxaliplatin and 2400 mg/m² of 5-fu). Sorafenib or donafenib is administered orally at 100-120 mg each time, twice a day.

When the disease progresses in any of the above treatment regimens as the treatment time is prolonged, the oral preparation of sorafenib or donafenib with low administration dosage of the present disclosure can be subjected to dose titration according to the clinical treatment results. For example, oral administration at 100-120 mg each time can be adjusted to 125-150 mg each time, 150-210 mg each time, 175-240 mg each time, 200-270 mg each time, 225-300 mg each time, 250-330 mg each time, 275-360 mg each time, or 300-400 mg each time, twice a day.

The oral preparation of sorafenib or donafenib with low administration dosage of the present disclosure can be used alone or in combination with any of the above-mentioned additional treatments, in order to prolong the overall survival and progression-free survival of patients, reduce the side effects caused by high drug exposure, and improve the tolerance and compliance of patients. The amount of the drug exposure is related to the therapeutic effect, but also related to some adverse reactions. The higher the exposure, the more severe hand foot skin reactions (HFSR). According to the patient's tolerance, the oral preparation of sorafenib or donafenib with low administration dosage of the present disclosure is first administered orally at 50 mg-60 mg for a certain period of time, and then the dose is titrated to 50 mg-60 mg, twice a day. After maintaining the dose for a period of time, according to the patient's tolerance, the dose is adjusted to 50 mg-60 mg each time, once a day for a period of time. When the patient suffers from severe adverse reactions and is unable to tolerate it anymore, the medication regimen can be adjusted to administration once every other day at 50 mg-60 mg, or adjusted periodically according to the disease progression and patient tolerance.

The oral preparation of sorafenib or donafenib with low administration dosage of the present disclosure can be used alone or in combination with any of the above-mentioned additional treatments, in order to prolong the overall survival and progression-free survival of patients, reduce the side effects caused by high drug exposure, and improve the tolerance and compliance of patients. Sorafenib or donafenib can be administered at 50-240 mg every day, once a day.

The self-assembled polymer material in the oral preparation of sorafenib or donafenib provided by the present application uses a compound of two specific polymer materials as the compounding self-assembled construction unit. The two self-assembled polymer materials are coordinated with the guest molecule sorafenib for regulation to prevent the original molecular packing of sorafenib or donafenib molecules due to intermolecular interactions, and reconstruct a new self-assembled system with the compounding self-assembled construction unit in the new microenvironment, so that the obtained oral preparation can reduce the premature metabolism or efflux of drugs by CYP3A4/A5, CYP2D6, CYP2C8 or P-gp of P450 enzymes in gastrointestinal mucosal epithelial cells during the absorption process, and also reduce the decreased absorption caused by rapid molecular packing due to the intermolecular interaction of sorafenib molecules after rapid dissolution of the drug from the composition at the molecular level. Therefore, it can be taken at a lower dose with safety, less incidence of side effects, low Cₘₐₓ and AUC₀₋ₜ variation, low weight/volume, rapid disintegration, good physical and chemical stability that it can remain stable even if the moisture content is up to 5%.

In order to further understand the present disclosure, the oral preparation of sorafenib or donafenib with low dosage provided by the present disclosure and its use will be described in detail in conjunction with examples, and the protection scope of the present disclosure is not limited by the following examples.

According to the chemical structure of sorafenib, the groups that can undergo intermolecular interactions, as shown in FIG. 2, are not related to the methyl group attached to the amide. Therefore, whether it is sorafenib or donafenib, the intermolecular interaction, and the type of the interaction, the strength of the interaction and the molecular recognition between sorafenib or donafenib and the effective groups of the polymer materials in the self-assembled construction unit are all consistent.

Therefore, the following is illustrated using sorafenib as an example, but it is applicable to all cases of donafinib.

### Example 1 Screening of self-assembled construction units

Sorafenib or sorafenib tosylate was weighed in appropriate amounts, dissolved in a small amount of dimethyl sulfoxide, and diluted with acetonitrile to prepare a high-concentration stock solution containing about 50 mg of sorafenib per 1 ml to ensure that sorafenib was dispersed in the solution at the molecular level.

A phosphate buffer solution with pH 6.8 containing 0.1% sodium dodecyl sulfate (SDS) was used as the basic medium (pH 6.8 phosphate +0.1% SDS), and the preparation of the basic medium was in accordance with the technical guidelines for dissolution testing of oral solid preparations. The polymer materials povidone (PVP) PVP K25, PVP K30 and PVP K90, copovidone (copolymer of vinyl pyrrolidone-vinyl acetate) such as PVP VA64, hydroxypropyl methylcellulose acetate succinate (HPMCAS) such as HPMCAS H type (22%-26% methoxy, 6%-10% hydroxypropoxy, 10%-14% acetyl, and 4%-8% succinyl) or its equivalent type, HPMCAS MG (21%-25% methoxy, 5%-9% hydroxypropoxy, 7%-11% acetyl, and 10%-14% succinyl) or its equivalent type, HPMCAS LG (20%-24% methoxy, 5%-9% hydroxypropoxy, 5%-9% acetyl, 14%-18% succinyl) or its equivalent type in particle or powder form, hydroxypropyl methylcellulose such as HPMCE5 or K100LV, polyethylene glycol such as PEG 8000, and Soluplus were weighed in appropriate amounts, and dissolved in pH 6.8 phosphate +0.1% SDS to prepare media with different concentrations for later use.

50 ml of the above-mentioned basic medium or the basic medium containing a certain mass percentage of polymer materials as self-assembled construction units were measured, placed into a 100 ml Erlenmeyer flask with a stopper, and incubated in a shaker at 37°C. 1.0 ml (1000 µg/mL) of sorafenib stock solution was added to each Erlenmeyer flask with a stopper respectively, dispersed evenly at 37°C under ultrasound, and then transferred in a shaker at 37°C. Samples were taken at 0.5 h, 1 h, 2 h, 3 h, 4 h and 6 h, and centrifuged at 37°C, 13000 rpm. Then an appropriate amount of the supernatant was taken and diluted in a diluent of 50% acetonitrile. The concentration of sorafenib still existing in a molecular state in the system constructed with media containing different self-assembled construction units was detected by HPLC, and the results are shown in Table 1 below.

The stereochemical structure and single-crystal structure of the sorafenib molecule are shown in FIG. 1, where the N4 hydrogen on the methylamine carboxylate and N3 on the pyridine easily form intramolecular hydrogen bonds, while the hydrogen on N1 and N2 of the urea group easily form intermolecular hydrogen bonds, hydrophobic effects and π-π stacking with O1 of the methylamine carboxylate of another molecule, causing sorafenib molecules rapidly form molecular packing through intermolecular interactions after dissolved in the solution, which finally leads to the precipitation from the solution due to the formation of a solid supramolecular self-assembled system, as shown in FIG. 2.

**Table 2. Concentration changes of sorafenib in solutions incubated for different times in different systems**

| Single polymer material as self-assembled building unit | | Sorafenib content (µg/ml) | | | | | |
|---|---|---|---|---|---|---|---|
| | | 0.5h | 1h | 2h | 3h | 4h | 6h |
| Basic medium - pH 6.8 phosphate buffer + 0.1% SDS | | 105.72 | 6.32 | 8.70 | 5.33 | 5.74 | 5.61 |
| 0.5%PVP K25 | / | 722.12 | 716.11 | 702.76 | 487.61 | 277.65 | 208.61 |
| 0.5%PVP K30 | / | 734.18 | 726.74 | 652.77 | 370.67 | 269.24 | 216.53 |
| 0.5%PVP K90 | / | 765.28 | 749.53 | 699.31 | 571.32 | 448.75 | 310.28 |
| 0.5%PVP VA64 | / | 784.25 | 775.80 | 755.80 | 432.22 | 313.83 | 244.15 |
| 0.5%Soluplus | / | 741.49 | 726.70 | 743.36 | 751.55 | 755.31 | 759.31 |
| 0.5%HPMC AS H | / | 627.47 | 618.64 | 631.27 | 642.96 | 633.21 | 634.32 |
| 0.5%HPMC AS M | / | 590.47 | 364.03 | 58.72 | 44.35 | 40.46 | 37.15 |
| 0.5%HPMC AS L | / | 353.97 | 55.22 | 30.04 | 27.17 | 25.78 | 24.11 |
| 0.5%HPMC E5 | / | 602.53 | 198.12 | 93.95 | 61.45 | 48.82 | 38.45 |
| 0.5%HPMC K100LV | / | 44.94 | 18.84 | 6.73 | 5.29 | 4.75 | 4.13 |

According to the above results and FIG. 1, compared with the basic medium (without self-assembled construction units), the polymer materials except for 0.5%-HPMC K100LV used as construction units individually had a certain regulatory effect on the molecular packing of sorafenib in the solution. However, as the incubation time increased, the thermal motion of molecules increased, the chance of forming hydrogen bonds between molecules increased, and the molecular packing increased with time. 0.5%-povidone (polymer of PVP K25-K90 and N-vinylpyrolidone), 0.5%-copovidone (PVPVA64), 0.5%-Soluplus, and 0.5%-HPMCAS H type (10%-14% acetyl and 4%-8% succinyl) all had good regulation effects on molecular packing of sorafenib that the concentration of sorafenib in the self-assembled system at 6 h was 208.61 µg/mL-759.31 µg/mL, significantly higher than that in the basic medium at 6 h (5.61 µg/mL). Compared with the basic medium, the self-assembled systems constructed with 0.5% Soluplus and 0.5% HPMCAS H had higher encapsulation rate for sorafenib, which was 75.9% and 63.4% respectively and could be increased by at least 100 times. In addition, the self-assembled systems constructed with 0.5% Soluplus and 0.5% HPMCAS H showed a slow rising trend of encapsulated drug over time, which was related to the competition between Soluplus and HPMCAS H-type and sorafenib molecules to form hydrogen bonds. Furthermore, the strong hydrophobicity played a decisive role. For the other povidone series and copovidone series, molecular packing of sorafenib molecules gradually occurred with the extension of incubation time, but the concentration of sorafenib in the self-assembled system at 6 h was still 34-55 times that in the basic medium at 6 h, and the regulatory effect of K90 with high molecular weight on sorafenib molecular packing was better than that of K25, K30 and VA64. It is speculated that the K90 with high viscosity limits the thermal motion of sorafenib molecules themselves and reduces the chance of forming intermolecular hydrogen bonds.

0.5%-copovidone (such as PVP VA64) is a copolymer of vinyl acetate-N-vinylpyrrolidinone, in which N-vinylpyrrolidinone accounts for about 60% of the copolymer and vinyl acetate accounts for about 40%. Therefore, the hydrophobicity of copovidone with the same mass fraction was better than that of povidone series. Also, the density of hydrogen acceptor provided by copovidone is different from that provided by povidone, and the oil-water partition coefficient LogP of sorafenib molecule is about 5.6, which is highly hydrophobic. Therefore, 0.5%-PVP VA64 is better than the low-viscosity K25 and K30 of the povidone series in regulating sorafenib molecular packing, but its ability to maintain the stability of the self-assembled system in the later stage is slightly worse than K90.

0.5%-polyvinyl caprolactam-polyvinyl acetate-polyethylene glycol graft copolymer (Soluplus), compared with copovidone, has 13% more hydrophilic group PEG6000, and the hydrophobicity of 59% vinyllactam group and 30% vinyl acetate group is better than polyvinylpyrrolidone and copovidone, that is, it provides hydrophobic interaction. The hydrophilic group competes with sorafenib molecules for hydrogen bonds. Therefore, the supramolecular self-assembled system constructed with Soluplus has the strongest ability to coordinately regulate the molecular packing of sorafenib, and the constructed supramolecular self-assembled system is the most stable and has the highest encapsulation rate.

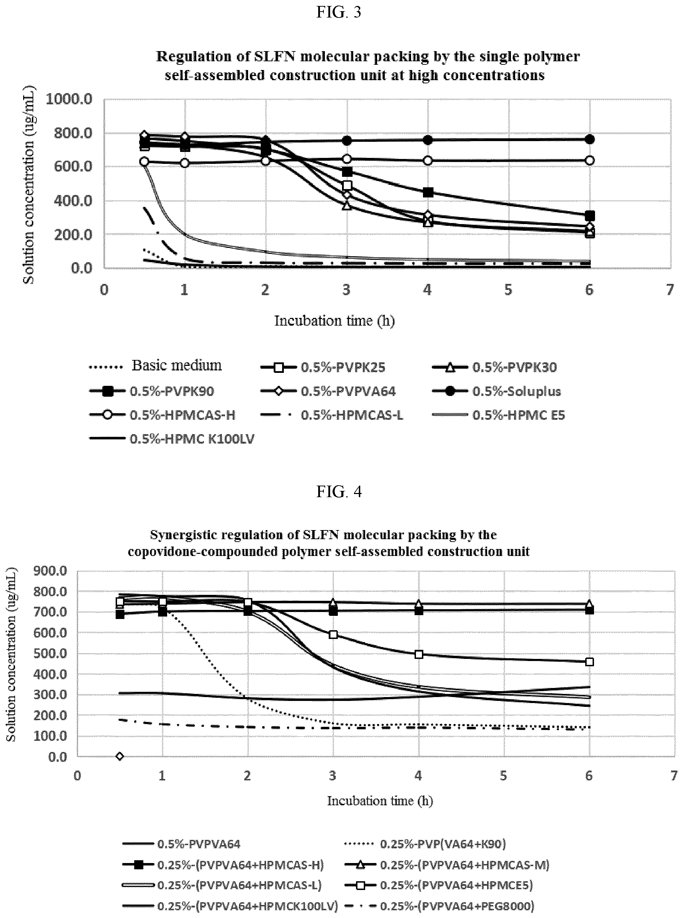

0.5%-Hydroxypropylmethylcellulose acetate succinate (HPMC AS) is a mixed ester of acetic acid and succinic acid of hydroxypropyl methylcellulose (HPMC). As the succinyl group increases in sequence and the acetyl group decreases in sequence, the hydrophobicity gradually weakens. There are generally three commercial specifications based on the substitution amount of succinyl and acetyl groups, H type (4%-8% succinyl and 10%-14% acetyl), L-type (14%-18% succinyl and 5%-9% acetyl), and M type in between. Hydroxypropyl methylcellulose acetate succinate can provide both hydrogen acceptor and hydrogen donor, and its succinyl group can also produce ionic interactions with weak alkaline drugs, which not only produce hydrophobic interactions with hydrophobic drugs, but also form a stable supramolecular self-assembled system with aqueous solutions under appropriate pH conditions. Therefore, its H-type has a stronger synergistic regulatory effect on molecule packing of sorafenib than L-type or M-type and can maintain stability for a long time. The details are shown in FIG. 3.

In summary, when sorafenib is prepared into a pharmaceutical composition at the molecular level, such as a solid dispersion, a solid solution, a polymer nanoparticle, a self-microemulsion, a nanoemulsion or a mixture at the molecular level, in order to solve the problem that the structure of the sorafenib molecule itself is easy to form a supramolecular self-assembled system and become solid, thereby affecting drug absorption, the polymer material selected as the supramolecular self-assembled construction unit needs to have a certain number of groups providing hydrogen acceptors and hydrogen donors, and furthermore, sufficient hydrophobic interaction and ionic interaction must be provided targeting the special structure and strong hydrophobic characteristics of the sorafenib molecule. Only in this way can the balance between the subsystem and the entire supramolecular system be maintained, so that more sorafenib molecules can be stably encapsulated in the newly constructed supramolecular self-assembled system for a long time.

However, each polymer material has its maximum safe daily dosage as a pharmaceutical adjuvant, especially for long-term administration. In addition, if the content of one polymer material in the formulation is too high (For a mass fraction of 0.5%, the unit preparation contains about 125 mg of adjuvants calculated based on 250 mL of intestinal juice. If two preparation units are to be administered each time, twice a day, the daily dosage of the carrier will be 500 mg/day, which is not achievable with many adjuvants), it will lead to changes in the preparation process, dissolution, disintegration behavior, physical stability, and chemical stability, often failing to achieve the expected results. Therefore, based on the above research, the inventors found that the compounding of two or more polymer materials with different properties as the construction unit of a supramolecular self-assembly system will achieve unexpected results. The specific scheme and experimental results are as follows

**Table 3. Sorafenib content of compounding self-assembled construction unit system after different times - table I**

| Compounding polymer self-assembled construction unit | | Sorafenib content (µg/ml) | | | | | |
|---|---|---|---|---|---|---|---|
| | | 0.5h | 1h | 2h | 3h | 4h | 6h |
| 0.25%PVP VA64 | 0.25%PVP K90 | 736.87 | 723.75 | 279.50 | 159.55 | 153.76 | 140.01 |
| | 0.25%HPMCAS H | 689.23 | 700.83 | 703.69 | 704.72 | 706.85 | 709.84 |
| | 0.25%HPMC AS M | 736.31 | 739.11 | 746.28 | 746.72 | 738.88 | 738.61 |
| | 0.25%HPMC AS L | 765.75 | 770.84 | 700.52 | 441.06 | 336.23 | 286.08 |
| | 0.25%HPMC E5 | 751.72 | 749.73 | 747.35 | 589.49 | 496.04 | 458.39 |
| | 0.25%HPMC K100LV | 305.70 | 308.36 | 280.88 | 273.87 | 288.30 | 335.26 |
| | 0.25%Soluplus | 746.80 | 736.34 | 748.31 | 727.40 | 741.31 | 738.56 |
| | 0.25%PEG 8000 | 176.67 | 154.98 | 141.27 | 135.78 | 138.33 | 129.16 |
| 0.25%PVP K25 | 0.25%PVP K90 | 718.47 | 728.57 | 736.70 | 732.90 | 734.47 | 629.50 |
| | 0.25%HPMC AS H | 694.67 | 692.52 | 706.99 | 696.97 | 725.35 | 710.76 |
| | 0.25%HPMC AS M | 720.06 | 711.52 | 687.53 | 620.63 | 516.31 | 403.80 |
| | 0.25%HPMC AS L | 715.39 | 700.47 | 620.13 | 471.35 | 366.58 | 304.92 |
| | 0.25%HPMC E5 | 708.37 | 677.84 | 432.13 | 391.52 | 403.22 | 357.77 |
| | 0.25%HPMC K100LV | 707.57 | 653.52 | 443.36 | 384.00 | 370.50 | 345.78 |
| | 0.25%Soluplus | 736.85 | 724.14 | 746.58 | 733.68 | 773.91 | 765.54 |
| | 0.25%PEG 8000 | 650.66 | 320.87 | 201.22 | 191.77 | 167.20 | 152.31 |
| 0.25%PVP K90 | 0.25%PVP K30 | 743.00 | 736.76 | 732.29 | 681.91 | 503.44 | 288.79 |
| | 0.25%HPMC AS H | 567.35 | 565.19 | 571.65 | 574.90 | 590.79 | 569.90 |
| | 0.25%HPMC AS M | 730.12 | 732.16 | 738.57 | 755.59 | 756.72 | 709.14 |
| | 0.25%HPMC AS L | 719.84 | 739.12 | 720.25 | 726.98 | 679.02 | 479.58 |
| | 0.25%HPMC E5 | 728.05 | 738.63 | 727.18 | 732.71 | 710.37 | 640.17 |
| | 0.25%HPMC K100LV | 755.35 | 743.98 | 751.79 | 743.42 | 664.69 | 492.40 |
| | 0.25%Soluplus | 699.53 | 693.61 | 716.05 | 694.25 | 703.20 | 702.38 |
| | 0.25%PEG 8000 | 741.38 | 725.55 | 716.11 | 543.88 | 387.87 | 281.67 |
| 0.25%Soluplus | 0.25%PVP K30 | 773.80 | 779.23 | 773.97 | 763.01 | 759.04 | 767.47 |
| | 0.25%HPMC AS M | 718.73 | 727.24 | 713.05 | 712.51 | 716.51 | 713.04 |
| | 0.25%HPMC E5 | 731.06 | 743.37 | 733.16 | 723.81 | 726.17 | 717.73 |
| | 0.25%HPMC K100LV | 774.76 | 778.00 | 775.83 | 772.29 | 773.43 | 683.04 |
| | 0.25%PEG 8000 | 767.86 | 777.41 | 768.78 | 750.34 | 734.58 | 546.93 |

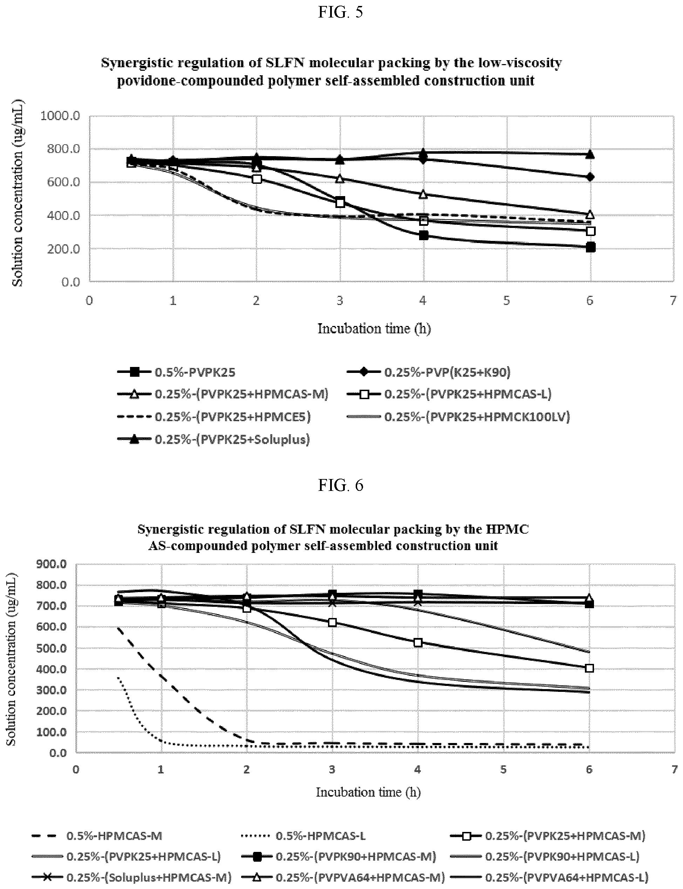

According to the above results, when the dosage of copovidone, povidone or Soluplus was reduced by half and compounded with other polymer materials with a mass ratio of 1:1, in addition to the significantly reduced daily dosage of single polymer material, an unexpected synergistic regulation effect was also found, as shown in FIGs. 4-7 for details.

Copovidone, taking PVP VA64 as an example, lacks hydrophilic groups and hydrogen donor groups compared with Soluplus or HPMCAS H type. In addition, compared with sorafenib, it provides weaker hydrophobicity than Soluplus. In addition to the characteristics of copovidone, the povidone series has worse hydrophobicity than copovidone. The combined use of 0.25%-(PVPVA64) and 0.25% HPMCAS H type or HPMCAS M type or Soluplus can significantly make up for the shortcomings of PVP VA64 alone, and the concentrations of sorafenib in the self-assembled system measured at 6 h were all above 700 µg/mL. Similarly, 0.25%-(PVP-VA64) compounded with hydrophilic HPMC also has a significant synergistic effect in regulating the molecular packing of sorafenib, and the concentration of sorafenib in the self-assembled system was maintained at 335-458 µg/mL after 6 h, significantly higher than the effect achieved by any single polymer material in the compounded carrier. 0.25% PPVVA64 compounded with 0.25% PEG8000 showed a significantly reduced synergistic regulation effect on the molecular packing of sorafenib compared with 0.5%-PVPVA64 due to the high hydrophilicity of PEG8000.

For the povidone series, PVP K25 was used as an example of low-viscosity povidone and PVP K90 as an example of high-viscosity povidone. The 0.25% (PVP+HPMCAS) and 0.25% (PVP+HPMC) series were significantly better than any polymer material with high mass fraction used alone, especially better than 0.5%-HPMCAS M and L or 0.5%-PVP VA64 or 0.5%-PVP or 0.5% HPMCE5 and 0.5% HPMC K100LV. Based on the hydrophilic and hydrophobic groups in the structure of each polymer material and their ability to provide hydrogen acceptors and donors, the compounding self-assembled construction units make up for the limitations of single polymer materials and commercially available polymer materials, and can be reasonably compounded according to the structure of drugs themselves and the types of intermolecular interactions. What is even more unexpected is that the combined use of low-viscosity PVPK25 or K30 and high-viscosity PVP K90 had a synergistic regulation effect on the molecular packing of sorafenib significantly better than the effect of a single polymer material. With 0.25%-PVP (K25+K90), sorafenib can be maintained at 718-630 µg/mL from 0.5 h to 6 h, while 0.5%-PVPK25 and 0.5%-PVP K90 used as self-assembled construction units had a sorafenib concentration of 209-310 µg/mL measured in the system at 6 h.

The effects of 0.25%-(PVP-VA64/PVP+HPMC) series used in the compounding self-assembled construction units were also significantly better than that of any polymer material with high mass fraction used alone. The ability of 0.5% HPMC to coordinately regulate the molecular packing of sorafenib was very poor, and there was basically no difference compared with the basic medium. However, the effect of compounding 0.25%-(PVP-VA64/PVP+HPMC) was significantly better than that of 0.5%-PVP VA64, 0.5%-PVP and 0.5%-HPMC. In the 0.25% (PVPVA64+HPMC) group, the compounding of the low-viscosity HPMC and high-viscosity HPMC with copovidone were significantly better after than those used alone, and the synergistic regulation effect of low-viscosity HPMC compounded with PVP VA64 on the molecular packing of sorafenib was significantly better than that of high-viscosity HPMC K100 LV. In the 0.25% (PVP+HPMC) group, the synergistic regulation effects of PVP K25 compounded with high-viscosity and low-viscosity HPMC were basically the same.

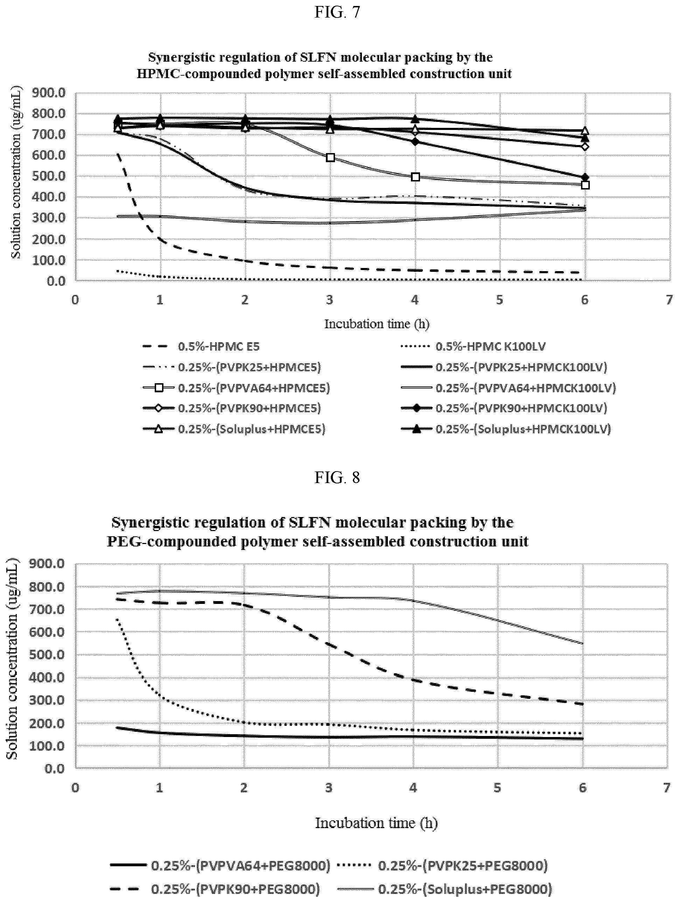

However, not all the compounding of polymer materials had a synergistic regulation effect on the molecular packing of sorafenib, such as the compounding of povidone, copovidone, and Soluplus with PEG8000, which was equivalent to the basic medium, as shown in FIG. 8.

Soluplus compounded with povidone, copovidone, HPMCAS series and HPMC series as the supramolecular self-assembled construction unit, under the premise that their mass fraction was reduced by half, still maintained the synergistic effect that can be achieved by the high mass fraction of Soluplus. It provides a new idea to solve the problems such as large amount of Soluplus, poor formability and the influence on the stability of active ingredients.

The compounding of 0.25%-PVP K90 and HPMCAS series or HPMC with low hydrophobicity group also showed significant synergistic regulation effects. The highly hydrophilic succinyl group of the HPMCAS series (L type and M type) provides ionic interaction for sorafenib, and can also compete with groups that easily form hydrogen bonds between sorafenib molecules, thereby destroying its original self-assembled system. PVP K90 makes up for the defects of HPMCAS (L type and M type) and HPMC caused by insufficient hydrophobicity, and also reduces the chance of molecular thermal motion and mutual contact, thereby reducing the formation of intermolecular hydrogen bonds. The effect of 0.25%-PVP K90+0.25%-HPMC K100LV also confirms this point, but the competition between the hydrogen donor or acceptor on the polymer material and the sorafenib molecule for hydrogen bonding and hydrophobicity is more significant.

Table 4 shows the performance of the supramolecular self-assembed system constructed with HPMCAS series (with different contents of hydrophobic acetyl group and hydrophilic succinyl group) compounded with other polymer materials.

**Table 4. Sorafenib content of different compounding self-assembled construction unit after different times - table II**

| Compounding self-assembled construction unit | | Sorafenib content (µg/ml) | | | | | |
|---|---|---|---|---|---|---|---|
| | | 0.5h | 1h | 2h | 3h | 4h | 6h |
| 0.25%HPMC AS HG | 0.25%PVP K30 | 807.81 | 790.92 | 731.13 | 774.00 | 800.26 | 818.59 |
| | 0.25%HPMC AS M | 844.47 | 778.77 | 731.46 | 693.66 | 457.77 | 242.80 |
| | 0.25%HPMC AS L | 775.58 | 687.81 | 331.79 | 219.41 | 188.27 | 148.39 |
| | 0.25%HPMC E5 | 803.58 | 753.96 | 668.02 | 485.32 | 345.18 | 264.42 |
| | 0.25%Soluplus | 788.00 | 772.02 | 777.24 | 825.22 | 798.02 | 793.68 |
| | 0.25%PVPVA64 | 758.46 | 769.04 | 776.38 | 773.45 | 787.20 | 777.54 |
| | 0.25%PEG 8000 | 660.69 | 623.36 | 309.80 | 183.02 | 160.04 | 123.47 |
| 0.25%HPMC AS L | 0.25%HPMC AS M | 694.89 | 635.12 | 677.09 | 653.06 | 645.55 | 725.87 |
| | 0.25%HPMC E5 | 724.56 | 694.99 | 288.91 | 226.62 | 284.41 | 314.20 |
| | 0.25%PVP K30 | 772.55 | 761.15 | 761.99 | 781.16 | 765.21 | 752.84 |
| | 0.25%PVPVA64 | 747.49 | 749.92 | 699.59 | 472.69 | 364.95 | 314.20 |
| | 0.25%Soluplus | 798.47 | 787.06 | 779.42 | 775.10 | 789.00 | 788.92 |
| 0.25%HPMCE5 | 0.5%PVP K25 | 774.69 | 781.58 | 779.78 | 783.86 | 767.96 | 624.66 |
| | 0.5%PVP VA64 | 783.35 | 783.51 | 770.08 | 577.76 | 480.02 | 455.29 |
| | 0.5%PVP K90 | 790.59 | 782.06 | 844.27 | 806.47 | 791.77 | 838.45 |
| | 0.5%PVP K30 | 785.23 | 783.85 | 775.76 | 778.48 | 778.43 | 613.82 |
| | 0.5%Soluplus | 786.65 | 786.29 | 794.47 | 780.43 | 792.36 | 823.19 |
| | 0.5%HPMC AS H | 752.49 | 746.23 | 744.13 | 740.41 | 731.24 | 690.65 |

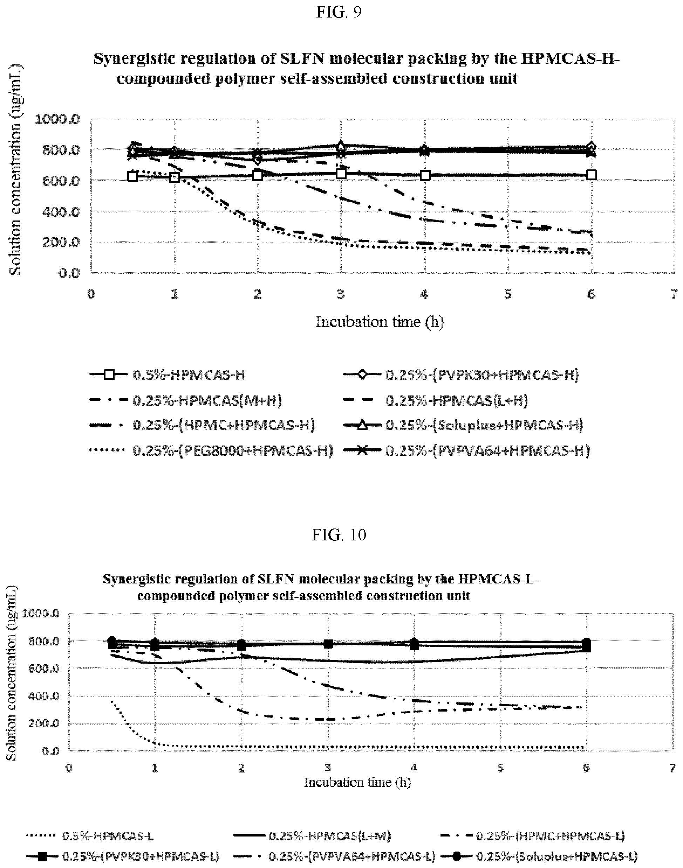

As can be seen from Table 4, the compounding of the HPMCAS series with other polymer materials showed significant selectivity. The compounding of 0.25%-HPMCAS H type (4%-8% succinyl group and 10%-14% acetyl group) with reduced mass fraction of povidone, copovidone, and Soluplus still maintained the high solubilization and crystal suppressing effect achieved at the original high mass fraction, and the concentration of sorafenib in the self-assembled system at 6 h was about 793-818 µg/mL, with an encapsulation rate up to 81.8%. When 0.25%-HPMCAS H type was compounded with an equal mass fraction of hydrophilic polymer materials, the synergistic regulation ability gradually decreased over time. After HPMCAS L type was compounded with the same mass fraction of HPMCAS M type, the constructed compounding self-assembled construction unit had hydrophobic acetyl groups of about 6%-10% and hydrophilic succinyl groups of about 12%-16%, and can achieve a synergistic regulation effect which was equivalent to that of HPMCAS H-type and far exceeded that of 0.5%-HPMCAS L-type or 0.5%-HPMCAS M-type when used alone. This is very important for the development of preparation products, and the characteristics of dissolution and release of the products in vivo could be adjusted according to the different ratios of the two. The compounding of HPMCAS L type with copolyvidone and povidone allows the two to complement each other's strengths and weaknesses, and the results are far beyond the effects of any single polymer material. The compounding of 0.25% HPMCAS L type and 0.25% Soluplus not only reduces the dosage of each, but also improves the feasibility of the preparation process. Meanwhile, it can control the drug release in vivo according to the designed release behavior. For the hydroxypropyl methylcellulose E series, the general methoxy group content is about 29%, and the hydroxypropyl group content is about 12%. The number of hydrogen donors and acceptors in its structure is higher than that of HPMCAS, and is more hydrophilic compared with HPMCAS. Therefore, its single use had no ability to regulate the formation of the molecular packing of sorafenib. However, when HPMCAS was compounded with the more hydrophobic povidone, copovidone, and Soluplus, the number of hydrogen donors and acceptors in the system was changed, and the constructed system was also made more hydrophobic. However, as the incubation time increased, e.g., for 6 hours, sorafenib packed slowly in the supramolecular system constructed with low-viscosity povidone, copovidone and HPMC, while high-viscosity povidone still showed slow growing until 6 hours. The compounding of 0.25%-HPMC (low viscosity) and the same mass fraction of povidone, copovidone, and high hydrophobic types of HPMCAS had a significant synergistic regulatory effect, which was better than the regulation effect that can be achieved by any one alone. The details are shown in FIGs. 9 and 10.

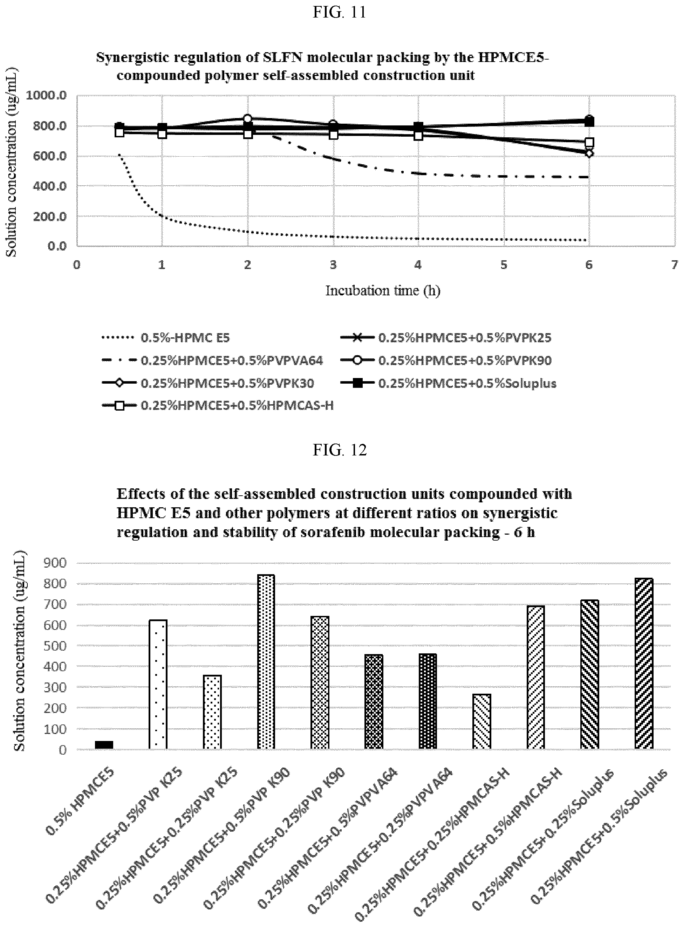

For HPMC, taking the E series as an example, the average content of methoxy group is about 29%, and the average content of hydroxypropyl group is 10%. Compared with HPMCAS, it has more hydrophilic groups and poor hydrophobicity. Therefore, increasing the mass fraction of the hydrophobic compounding crystallization inhibitor, such as 1:2, can significantly improve its solubilization and crystal inhibition ability. Among them, when 0.5% povidone, HPMCAS-H and 0.25% HPMCE5 were compounded, increasing the hydrophobic adjuvants can enhance the dissolution of sorafenib and inhibit the crystallization, but 0.5% copovidone and Soluplus did not show more obvious advantages, see FIGs. 11 and 12 for details.

The HPLC analysis method is as follows:

Agilent1260 high performance liquid chromatography, quaternary low pressure gradient pump, column oven, autosampler, ultraviolet detector and on-line degasser were used, and the parameters are shown in Table 5;

**Table 5. HPLC analysis method and parameters**

| | | | |
|---|---|---|---|
| Column: | Octadecylsilane bonded silica gel as filler (Waters CORTECS C18, 4.6×50 mm; 2.7 µm) | | |
| Mobile phase: | Mobile phase A: 0.1% H3PO4 solution | | |
| | Mobile phase B: acetonitrile | | |
| Gradient elution: | Gradient Elution Table | | |
| | Time/min | Mobile phase A/% | Mobile phase B/% |
| | 0 | 70 | 30 |
| | 5 | 0 | 100 |
| | 5.1 | 70 | 30 |
| | 10 | 70 | 30 |
| Flow rate: | 1.0ml/min | | |
| Column temperature: | 35°C | | |
| Wavelength: | 262nm | | |
| Injection volume: | 10µl | | |

### Example 2 Preparation of pharmaceutical compositions at molecular level with low amounts of carrier

Sorafenib (SLFN)/ Sorafenib tosylate (TSSLFN) was added into a mixed solvent of methanol-dichloromethane (1:3) and dissolved under stirring at 30-33°C with a ratio of the main drug to the mixed solvent of 1:100. Then a carrier was added and dissolved at a ratio of the main drug to the carrier of 1:1. Spray drying was performed, wherein the inlet air temperature was 120°C, the outlet air temperature was set at 60°C, and nitrogen was used as carrier gas.

**Table 6. DSC detection results of molecular-level pharmaceutical compositions prepared with different carriers and different ratios of carriers to main drug**

| Main drug | Carrier | Ratio | Endothermic peaks of the crystalline drug in the DSC curve | Endothermic peaks of the crystalline drug in the DSC curve after 10 days at 40°C /RH75% |
|---|---|---|---|---|
| SLFN | None | -- | at 208-220°C | / |
| SLFN | PVP K30 | 1:1 | None | None |
| SLFN | HPC LF | 1:1 | Yes | / |
| SLFN | HPMC E5 | 1:1 | None | Yes |
| SLFN | VA64 | 1:1 | None | None |
| SLFN | PVP K25+K90 | 1:0.8:0.2 | Yes | / |
| SLFN | PVP K25+VA64 | 1:0.5:0.5 | Yes | / |
| SLFN | PVP K30+HPMCAS L | 1:0.85:0.15 | Yes | / |
| SLFN | PVPK90+HPMCAS | 1:0.85:0.15 | Yes | / |
| SLFN | Soluplus | 1:1 | None | Yes |
| SLFN | VA64+HPMCAS | 1:1:0.15 | None | None |
| TSSLFN | None | -- | at 180-200°C and/or 215-235°C | / |
| TSSLFN | VA64 | 1:1 | None | None |

According to the above results, under low carrier ratio conditions, the pharmaceutical compositions at the molecular level can be formed using copovidone, povidone, copovidone+HPMCAS, HPMC E5, and Soluplus as carriers.

### Example 3 Tablet Sample Preparation

Preparation process: The pharmaceutical compositions at the molecular level (SD) were prepared according to Example 2. The SD, self-assembled construction units, half of the formulation amount of a filler, a disintegrant, and half of the formulation amount of a lubricant were mixed well, subjected to dry granulation, added with the remaining half of the formulation amount of the filler, a glidant, and half of the formulation amount of the lubricant, mixed well, and pressed into tablets. The formulations of the tablet samples are shown in Table 7.

**Table 7 Formulations of tablet samples (mg/tablet)**

| Formulations | Pharmaceutical compositions at the molecular level | | | Self-assembled construction units | | | |
|---|---|---|---|---|---|---|---|
| | SLFN | VA64 | HPMCAS | PVPK30 | HPMC E5 | HPMCAS H | HPMCAS M |
| F1 | 50 | 50 | / | 10 | 10 | / | / |
| F2 | 50 | 50 | / | 20 | 5 | / | / |
| F3 | 50 | 50 | / | 20 | 20 | / | / |
| F4 | 50 | 50 | / | 15 | 15 | / | / |
| F5 | 50 | 50 | / | 20 | / | 10 | / |
| F6 | 50 | 50 | / | 10 | / | 15 | / |
| F7 | 50 | 50 | / | 10 | / | / | / |
| F8 | 50 | 50 | / | 10 | / | / | / |
| F9 | 50 | 50 | / | / | 15 | 15 | / |
| F10 | 50 | 50 | / | / | 15 | / | / |
| F11 | 50 | 50 | / | / | 10 | / | / |
| F12 | 50 | 50 | / | / | 15 | / | / |
| F13 | 50 | 50 | / | / | 10 | / | / |
| F14 | 50 | 50 | / | / | / | 15 | / |
| F15 | 50 | 50 | / | / | / | 15 | / |
| F16 | 50 | 50 | / | / | / | 15 | / |
| F17 | 50 | 50 | / | / | / | 15 | / |
| F18 | 50 | 50 | / | / | / | 20 | / |
| F19 | 50 | 50 | / | / | / | 10 | / |
| F20 | 50 | 50 | / | / | / | / | 15 |
| F21 | 50 | 50 | / | / | / | / | 20 |
| F22 | 50 | 50 | / | / | / | / | 10 |
| F23 | 50 | 50 | / | / | / | / | 15 |
| F24 | 50 | 50 | / | / | / | / | / |
| F25 | 50 | 50 | / | / | / | / | / |
| F26 | 50 | 50 | / | / | / | / | / |
| F27 | 50 | 50 | / | / | / | / | / |
| F28 | 50 | 50 | / | / | / | / | / |
| F29 | 50 | 50 | / | / | / | / | / |
| F30 | 50 | 50 | / | / | / | / | / |
| F31 | 50 | 50 | / | / | / | / | / |
| F32 | 50 | 50 | / | / | / | / | / |
| F33 | 50 | 50 | 15 | 40 | 10 | / | / |
| F34 | 50 | 50 | 15 | / | 10 | 30 | / |
| F35 | 50 | 50 | 15 | / | 20 | 10 | / |
| F36 | 50 | 50 | 15 | / | 15 | / | / |
| F37 | 50 | 50 | 15 | 20 | / | / | / |
| F38 | 50 | 50 | 15 | / | 15 | / | / |
| F39 | 50 | 50 | 15 | / | / | 15 | / |
| F40 | 50 | 80 | 10 | / | / | 10 | / |
| F41 | 50 | 60 | 15 | 25 | 25 | / | / |
| F42 | 50 | 40 | 10 | 20 | / | 25 | / |
| F43 | 50 | 150 | / | 30 | / | / | / |
| DB1 | 50 | 50 | 15 | / | / | / | / |
| F1 | / | / | / | / | / | / | / |
| F2 | / | / | / | / | / | / | / |
| F3 | / | / | / | / | / | / | / |
| F4 | / | / | / | / | / | / | / |
| F5 | / | / | / | / | / | / | / |
| F6 | / | / | / | / | / | / | / |
| F7 | 20 | / | / | / | / | / | / |
| F8 | / | / | / | / | 20 | / | / |
| F9 | / | / | / | / | / | / | / |
| F10 | / | 25 | / | / | / | / | / |
| F11 | / | / | 15 | / | / | / | / |
| F12 | / | / | / | 20 | / | / | / |
| F13 | / | / | / | / | 15 | / | / |
| F14 | / | / | / | / | / | / | / |
| F15 | / | 20 | / | / | / | / | / |
| F16 | / | 15 | / | / | / | / | / |
| F17 | / | / | 15 | / | / | / | / |
| F18 | / | / | / | 10 | / | / | / |
| F19 | / | / | / | / | 10 | / | / |
| F20 | 15 | / | / | / | / | / | / |
| F21 | / | 15 | / | / | / | / | / |
| F22 | / | / | / | / | 15 | / | / |
| F23 | / | / | 10 | / | / | / | / |
| F24 | 20 | / | 10 | / | / | / | / |
| F25 | 10 | / | / | / | 10 | | / |
| F26 | / | 20 | / | / | 15 | / | / |
| F27 | / | / | 10 | 15 | / | / | / |
| F28 | / | / | 10 | / | / | / | 15 |
| F29 | / | / | 10 | / | 15 | / | / |
| F30 | / | / | / | 15 | 10 | / | / |
| F31 | / | / | / | / | 10 | / | 10 |
| F32 | / | / | / | / | 15 | 20 | / |
| F33 | / | / | / | / | / | / | / |
| F34 | / | / | / | / | / | / | / |
| F35 | / | / | / | / | / | / | / |
| F36 | / | / | 10 | / | / | / | / |
| F37 | / | / | / | / | 20 | / | / |
| F38 | / | / | / | 20 | / | / | / |
| F39 | / | / | 10 | / | / | / | / |
| F40 | / | / | / | / | 5 | / | / |
| F41 | / | / | / | / | / | / | / |
| F42 | / | / | / | / | / | / | / |
| F43 | / | / | / | / | / | / | / |
| DB1 | / | / | / | / | / | / | / |

**Table 8. Formulations of tablets - amounts of other adjuvants**

| Adjuvants | Filler | Disintegrant | Glidant | Lubricant |
|---|---|---|---|---|
| | SMCC | CCNa | SiO2 | MS |
| Amount | 121 | 22.5 | 15 | 3 |

Dissolution detection: paddle method, 900 mL of pH 6.8 phosphate buffer solution containing 0.1% sodium dodecyl sulfate (SDS), 100 rpm, 37°C. Sampling and sample treatment: 10 mL of the sample was taken and filtered with PES filter membrane (diameter of 25 mm, pore size of 0.45 µm). 8 mL of filtrate was discarded. 1-2 mL of the remaining filtrate was taken, and 10 mL of medium was added in the dissolution cup. The filtered sample was sent to analysis and detected after dilution. 1 tablet (200 mg) of reference list drug (RLD) and 4 tablets (200 mg) of the preparations of the present disclosure were used for detection. The dissolution detection was carried out by HPLC and the dissolution results are shown in the table below.

**Table 9. Dissolution results of tablet formulations**

| Formulations | Cumulative Dissolution (%) | | | | |
|---|---|---|---|---|---|
| | 5min | 15min | 30min | 60min | 120min |
| F1 | 81 | 65 | 41 | 28 | 22 |
| F2 | 87 | 74 | 53 | 39 | 31 |
| F3 | 86 | 78 | 54 | 39 | 32 |
| F4 | 80 | 80 | 56 | 40 | 31 |
| F5 | 83 | 81 | 57 | 43 | 33 |
| F6 | 87 | 74 | 51 | 36 | 29 |
| F7 | 80 | 75 | 57 | 42 | 34 |
| F8 | 79 | 68 | 51 | 34 | 29 |
| F9 | 87 | 82 | 50 | 36 | 30 |
| F10 | 89 | 72 | 49 | 38 | 31 |
| F11 | 73 | 85 | 58 | 43 | 36 |
| F12 | 89 | 78 | 54 | 43 | 34 |
| F13 | 83 | 74 | 43 | 34 | 27 |
| F14 | 55 | 42 | 37 | 26 | 19 |
| F15 | 88 | 74 | 46 | 39 | 29 |
| F16 | 83 | 80 | 51 | 43 | 37 |
| F17 | 83 | 76 | 49 | 39 | 30 |
| F18 | 77 | 74 | 45 | 31 | 26 |
| F19 | 81 | 64 | 50 | 33 | 21 |
| F20 | 64 | 71 | 45 | 30 | 22 |
| F21 | 83 | 61 | 47 | 35 | 29 |
| F22 | 79 | 70 | 42 | 30 | 22 |
| F23 | 83 | 75 | 47 | 36 | 29 |
| F24 | 75 | 58 | 41 | 24 | 17 |
| F25 | 82 | 65 | 44 | 34 | 27 |
| F26 | 82 | 60 | 41 | 30 | 21 |
| F27 | 89 | 82 | 63 | 47 | 38 |
| F28 | 79 | 77 | 52 | 37 | 29 |
| F29 | 83 | 76 | 55 | 39 | 31 |
| F30 | 81 | 72 | 51 | 40 | 33 |
| F31 | 73 | 68 | 43 | 30 | 21 |
| F32 | 79 | 76 | 50 | 39 | 31 |
| F33 | 76 | 65 | 41 | 27 | 20 |
| F34 | 81 | 71 | 50 | 37 | 29 |
| F35 | 76 | 74 | 55 | 42 | 34 |
| F36 | 73 | 70 | 52 | 40 | 33 |
| F37 | 70 | 75 | 54 | 43 | 36 |
| F38 | 71 | 69 | 48 | 39 | 31 |
| F39 | 81 | 65 | 46 | 37 | 29 |
| F40 | 74 | 60 | 40 | 32 | 24 |
| F41 | 83 | 80 | 56 | 43 | 34 |
| F42 | 86 | 81 | 52 | 43 | 35 |
| F43 | 40 | 45 | 32 | 19 | 17 |
| DB1 | 70 | 36 | 27 | 15 | 15 |
| RLD | 7 | 7 | 7 | 6 | 5 |

It can be seen from the above results that the cumulative dissolution rates of formulations F1 to F42 were much higher than that of RLD, F43 (a molecular-level pharmaceutical composition with low main drug content), and DB1. In addition, the compounding self-assembled construction units in the preparation had a better synergistic regulation effect on the molecular packing of sorafenib.

### Example 4 Investigation of tablet hardness, disintegration and preparation stability by tablet adjuvants and moisture

Preparation process: The solid dispersions (SD) were prepared according to Example 2. The SD, crystallization inhibitor, half of the formulation amount of a filler, a disintegrant, and half of the formulation amount of a lubricant were mixed well, subjected to dry granulation, added with the remaining half of the formulation amount of the filler, a glidant, and half of the formulation amount of the lubricant, mixed well, and pressed into tablets. Moisture measurement was performed according to the first method of 0831, Four General Chapters of the Chinese Pharmacopoeia 2020 Edition.

The formulations for tablet preparation are shown in the table below.

**Table 10. Formulations of different adjuvants for tablets**

| Formulations | SD | | Crystallization inhibitor | | Filler | | | |
|---|---|---|---|---|---|---|---|---|
| | SLFN | VA6 4 | PVPK30 | HPMC E5 | SMCC | MCC101 | Lactose | Mannitol |
| F44 | 50 | 50 | 25 | 15 | 118 | / | / | / |
| F45 | 50 | 50 | 25 | 15 | / | 118 | / | / |
| F46 | 50 | 50 | 25 | 15 | / | / | 118 | / |
| F47 | 50 | 50 | 25 | 15 | / | / | / | 118 |
| F48 | 50 | 50 | 25 | 15 | 118 | / | / | / |
| F49 | 50 | 50 | 25 | 15 | 118 | / | / | / |
| F50 | 50 | 50 | 25 | 15 | 100 | / | / | / |
| F51 | 50 | 50 | 25 | 15 | 90 | / | / | / |
| F52 | 50 | 50 | 25 | 15 | 150 | / | / | / |
| F53 | 50 | 50 | 25 | 15 | 150 | / | / | / |
| F54 | 50 | 50 | 25 | 15 | 100 | / | / | / |
| F55 | 50 | 50 | 25 | 15 | 110 | / | / | / |
| F56 | 50 | 50 | 25 | 15 | 110 | / | / | / |
| F57 | 100 | 100 | 50 | 30 | 220 | / | / | / |

| Formulations | Disintegrant | | | Glidant | Lubricant | | Moisture (%) |
|---|---|---|---|---|---|---|---|
| | CCNa | PVPP | CMS-Na | SiO₂ | MS | Sodium stearyl fumarate | |
| F44 | 24 | / | / | 15 | 3 | / | 5.1 |
| F45 | 24 | / | / | 15 | / | / | 5.0 |
| F46 | 24 | / | / | 15 | / | / | 5.1 |
| F47 | 24 | / | / | 15 | / | / | 4.7 |
| F48 | / | 24 | / | 15 | / | / | 4.4 |
| F49 | / | / | 24 | 15 | 3 | / | 4.2 |
| F50 | 24 | / | / | 15 | / | 3 | 4.0 |
| F51 | 24 | / | / | 15 | 3 | / | 3.5 |
| F52 | 24 | / | / | 15 | 3 | / | 4.1 |
| F53 | 17 | / | / | 15 | 3 | / | 3.7 |
| F54 | 45 | / | / | 15 | 3 | / | 4.1 |
| F55 | 30 | / | / | 15 | 3 | / | 4.3 |
| F56 | 24 | / | / | 10 | 3 | / | 2.8 |
| F57 | 48 | / | / | 30 | 6 | / | 2.9 |

Hardness: A tablet hardness tester was used for testing, in which 6 tablets were tested, and the mean value was taken. Disintegration time: A disintegration tester was used for testing, in which 6 tablets were tested, and the mean value was taken. CTF-aniline and total impurities: detected by HPLC. Crystal content: detected by Raman spectroscopy. The results are shown in Table 10. The stability test was carried out in an accelerated test chamber at 40°C±2°C /RH75%±5%. The tablets for stability investigation were packed in aluminum-plastic composite film plus double aluminum packaging.

**Table 11. Investigation of tablet hardness, disintegration and stability by tablet adjuvants and moisture**

| Formulations | Hardness (N) | Disintegration time (min) | CTF-aniline¹ (ppm) | | Total impurities (%) | | Crystal content² (%) | |
|---|---|---|---|---|---|---|---|---|
| | | | Day 0 | 60°C for 10 days | Day 0 | 60°C for 10 days | Day 0 | 40°C /RH75% for 90 days |
| F44 | 63 | 2.5 | Not detected | Not detected | 0.235 | 0.266 | Not detected | Not detected |
| F45 | 54 | 3.3 | Not detected | Not detected | 0.231 | 0.274 | Not detected | Not detected |
| F46 | 32 | 8.9 | Not detected | Not detected | 0.209 | 0.281 | Not detected | Detected |
| F47 | 30 | 10.4 | Not detected | Not detected | 0.211 | 0.275 | Not detected | Detected |
| F48 | 55 | 7.9 | Not detected | Not detected | 0.212 | 0.261 | Not detected | Detected |
| F49 | 57 | 14.3 | Not detected | Not detected | 0.228 | 0.259 | Not detected | Detected |
| F50 | 54 | 3.8 | Not detected | Not detected | 0.247 | 0.269 | Not detected | Not detected |
| F51 | 50 | 4.3 | / | / | / | / | / | / |
| F52 | 72 | 2.9 | / | / | / | / | / | / |
| F53 | 75 | 4.1 | / | / | / | / | / | / |
| F54 | 52 | 1.5 | / | / | / | / | / | / |
| F55 | 61 | 2.1 | / | / | / | / | / | / |
| F56 | 59 | 3.1 | / | / | / | / | / | / |
| F57 | 79 | 3.7 | / | / | / | / | / | / |

It can be seen from the above data that tablet fillers microcrystalline cellulose and silicified microcrystalline cellulose were significantly better than lactose and mannitol, and the amount range was 30-45% of the total weight of the tablet. The disintegrant was preferably croscarmellose sodium, and the amount accounted for 5-15% of the total weight of the tablet. The tablet also contained a lubricant, preferably magnesium stearate or sodium stearyl fumarate, more preferably magnesium stearate. The preparation prepared based on the preferred formulation can disintegrate rapidly within 5 minutes.

According to the above data, under high moisture conditions, tablets with microcrystalline cellulose or silicified microcrystalline cellulose as the filler and croscarmellose sodium as the disintegrant can be used. Sorafenib tablets did not experience molecular packing after 90 days at 40°C/RH 75% with packaging.

### Example 5 Preparation of coated tablets

The polymer materials of the self-assembled construction units can be added during the preparation of the tablet core, or added to the coating material, or can be added into the both. Preparation process: The pharmaceutical compositions at the molecular level (SD) were prepared according to Example 2. The SD, the polymer materials of the self-assembled construction units, half of the formulation amount of a filler, a disintegrant, and half of the formulation amount of a lubricant were mixed well, subjected to dry granulation, added with the remaining half of the formulation amount of the filler, a glidant, and half of the formulation amount of the lubricant, mixed well, pressed into tablets, and coated. Tablets were prepared according to the formulations in the following table.

**Table 12 Formulations of coated tablets**

| F | | SD | | | Self- | | G1 | Fill | Di | L | | Self-assembled construction units added to | | | | Other | |
|---|---|---|---|---|---|---|---|---|---|---|---|---|---|---|---|---|---|
| o r m ul at io n s | | | assembled construction units in the tablet core | | id an t | er | sin teg ran t | u b ri c a n t | the coating liquid | | | | | | | ingredient of the coating | |
| | SL FN | V A6 4 | PVP K30 | Solu plus | Si O 2 | SM CC | C C Na | M S | HP MC E5 | HP MC AS H | HP MC AS M | HP MC AS L | V A6 4 | P V P K3 0 | Sol upl us | Tita niu m Dio xid e | Trie thyl Citr ate |
| F 5 8 | 50 | 50 | 25 | / | 15 | 11 8 | 24 | 3 | 15 | / | / | / | / | / | / | 10 | 4 |
| F 5 9 | 50 | 50 | / | 10 | 15 | 11 8 | 24 | 3 | 15 | / | / | / | / | / | 10 | 10 | 4 |
| F 6 0 | 50 | 50 | / | / | 15 | 11 8 | 24 | 3 | 10 | 10 | / | / | / | / | / | 10 | 4 |
| F 6 1 | 50 | 50 | / | / | 15 | 11 8 | 24 | 3 | 15 | / | / | / | / | 15 | / | 10 | 4 |
| F 62 | 50 | 50 | / | / | 15 | 11 8 | 24 | 3 | / | 15 | / | 15 | / | / | / | 10 | 4 |
| F 6 3 | 50 | 50 | / | / | 15 | 11 8 | 24 | 3 | / | 10 | 20 | / | / | / | / | 10 | 4 |
| F 6 4 | 50 | 50 | / | / | 15 | 11 8 | 24 | 3 | / | / | / | 15 | / | / | 15 | 10 | 4 |
| F 6 5 | 50 | 50 | / | / | 15 | 11 8 | 24 | 3 | / | / | 10 | / | 10 | / | / | 10 | 4 |

Dissolution detection: paddle method, 900 mL of pH 6.8 phosphate buffer solution containing 0.1% sodium dodecyl sulfate (SDS), 100 rpm, 37°C. Sampling and sample treatment: 10 mL of the sample was taken and filtered with PES filter membrane (diameter of 25 mm, pore size of 0.45 µm). 8 mL of filtrate was discarded. 1-2 mL of the remaining filtrate was taken, and 10 mL of medium was added in the dissolution cup. After filtration, the samples were diluted in equal proportions and then tested. 1 tablet (200 mg) of reference list drug (RLD) and 4 tablets (200 mg) of the preparations of the present disclosure were used for detection. The dissolution results are shown in the table below.

**Table 13 Dissolution results of coated tablets**

| Formulations | Cumulative Dissolution (%) | | | | |
|---|---|---|---|---|---|
| | 5min | 15min | 30min | 60min | 120min |
| F58 | 81 | 79 | 52 | 39 | 29 |
| F59 | 83 | 74 | 48 | 36 | 28 |
| F60 | 85 | 76 | 52 | 38 | 31 |
| F61 | 77 | 78 | 53 | 40 | 33 |
| F62 | 84 | 81 | 53 | 42 | 26 |
| F63 | 82 | 80 | 55 | 41 | 33 |
| F64 | 85 | 80 | 48 | 34 | 27 |
| F65 | 86 | 78 | 50 | 38 | 28 |

From the above results, it can be seen that whether the self-assembled construction polymer material was added to the tablet core or the coating, or added to the both, all showed significant effects.

### Example 6 Preparation of pellets

Preparation process: Sorafenib and VA64 were mixed and dissolved in dichloromethane and methanol (3:1), layered with the drug using a fluidized bed, and then coated with a coating layer by spraying to obtain pellets. The resulting pellets were put into capsules, and each capsule contained 50 mg of sorafenib. Pellets were prepared according to the formulations below.

**Table 14 Formulations for pellets (mg)**

| Formulations | Blank pill core | Drug-containing layer | | Coating layer | | | | |
|---|---|---|---|---|---|---|---|---|
| | | SLFN | VA64 | HPMC E5 | HPMCAS H | Soluplus | Tal c | Triethyl Citrate |
| F66 | 50 | 50 | 50 | 15 | 15 | / | 10 | 4 |
| F67 | 50 | 50 | 50 | 15 | / | 10 | 10 | 4 |
| F68 | 50 | 50 | 50 | 10 | 5 | 5 | 10 | 4 |

Dissolution detection: paddle method, 900 mL of pH 6.8 phosphate buffer solution containing 0.1% sodium dodecyl sulfate (SDS), 100 rpm, 37°C. Sampling and sample treatment: 10 mL of the sample was taken and filtered with PES filter membrane (diameter of 25 mm, pore size of 0.45 µm). 8 mL of filtrate was discarded. 1-2 mL of the remaining filtrate was taken, and 10 mL of medium was added in the dissolution cup. After filtration, the samples were diluted in equal proportions and then tested. 1 tablet (200 mg) of reference list drug (RLD) and 4 tablets (200 mg) of the preparations of the present disclosure were used for detection. The dissolution results are shown in the table below.

**Table 15. Dissolution results of coated tablets**

| Formulations | Cumulative Dissolution (%) | | | | |
|---|---|---|---|---|---|
| | 5min | 15min | 30min | 60min | 120min |
| F66 | 73 | 61 | 43 | 39 | 29 |
| F67 | 76 | 64 | 48 | 38 | 30 |
| F68 | 69 | 73 | 52 | 38 | 31 |

It can be seen from the above results that the pellets were prepared as molecular-level pharmaceutical compositions by co-precipitation method, and coated with polymer materials containing the self-assembled construction units, which also had obvious synergistic regulation of the molecular packing of sorafenib after dissolution.

### Example 7 Powder coating

The pharmaceutical compositions of sorafenib at the molecular level were prepared according to the spray drying method in Example 2. The pharmaceutical compositions (SD) were coated with powder according to the formulation in the following table, and then added with adjuvants according to the formulation in Table 8 to prepare tablets. The powder coating was prepared according to the formulation in the table below.

**Table 16. Formulations for powder coating (mg)**

| Formulations | SD | | | Coating layer | | | | |
|---|---|---|---|---|---|---|---|---|
| | SLFN | VA6 4 | HPMCAS H | PVPK25 | HPMC E5 | Soluplus | Tal c | Triethyl Citrate |
| F69 | 50 | 50 | 15 | 15 | 15 | / | 10 | 4 |
| F70 | 50 | 50 | 15 | / | 15 | 10 | 10 | 4 |
| F71 | 50 | 50 | 15 | 5 | 15 | 10 | 10 | 4 |

Dissolution detection: paddle method, 900 mL of pH 6.8 phosphate buffer solution containing 0.1% sodium dodecyl sulfate (SDS), 100 rpm, 37°C. Sampling and sample treatment: 10 mL of the sample was taken and filtered with PES filter membrane (diameter of 25 mm, pore size of 0.45 µm). 8 mL of filtrate was discarded. 1-2 mL of the remaining filtrate was taken, and 10 mL of medium was added in the dissolution cup. The filtered sample was sent to analysis and detected after dilution. 1 tablet (200 mg) of reference list drug (RLD) and 4 tablets (200 mg) of the preparations of the present disclosure were used for detection. The dissolution results are shown in the table below.

**Table 17. Dissolution results of coated tablets**

| Formulations | Cumulative Dissolution (%) | | | | |
|---|---|---|---|---|---|
| | 5min | 15min | 30min | 60min | 120min |
| F69 | 82 | 76 | 53 | 39 | 33 |
| F70 | 80 | 80 | 61 | 42 | 35 |
| F71 | 79 | 78 | 54 | 40 | 37 |

It can be seen from the above results that SD was powder-coated with a coating material containing the self-assembled construction units. Sorafenib entered the solution after dissolution, and the self-assembled polymer material in the coating layer had entered the system in the outermost layer, which still showed a good synergistic regulatory effect on the molecular packing of sorafenib.

### Example 7 Preparation of self-microemulsion

Ethyl oleate, polyoxyethylene castor oil and ethanol were mixed well in proportion to form a blank self-microemulsion. Sorafenib was added and dissolved, and then the self-assembed construction units were added and mixed well. Self-microemulsions were prepared according to the following table.

**Table 18. Formulations of self-microemulsion (mg)**

| Formulations | SLFN | Ethyl oleate | Polyoxyethylene Castor Oil | Ethanol | PVPK30 | HPMCASH | Soluplus |
|---|---|---|---|---|---|---|---|
| F72 | 50 | 200 | 400 | 350 | 20 | 15 | / |
| F73 | 50 | 200 | 400 | 350 | 15 | / | 10 |
| F74 | 50 | 200 | 400 | 350 | / | 10 | 30 |
| F75 | 50 | 200 | 400 | 350 | / | / | / |

### Lipolysis test:

Lipolysis buffer: Tris maleate buffer (0.474 g/L), anhydrous calcium chloride (0.208 g/L), NaCl aqueous solution (8.766 g/L, ultrapure water), and 5 mol/L NaOH to adjust the pH to 6.5. Lipolysis medium: L-α-phosphatidylcholine (PC) (0.576 g/L). Sodium taurodeoxycholate (1.565 g/L) was added to the buffer, and the medium was stirred overnight to ensure that PC was completely dissolved. Pancreatin solution: 3 g of ground pig pancreatic juice was added to 15 ml lipolysis buffer, and 5 mol/L of NaOH was used to adjust the pH to 6.5. The mixture was stirred with a magnetic stirrer for 10 min, divided into equal parts, and then centrifuged (5°C, 10 min). The supernatant was taken for lipolysis experiment. Detection of trypsin enzyme activity: Before the lipolysis experiment began, the enzyme activity of trypsin was determined by using tributyrin as the enzyme substrate. An excess of tributyrin was added to ensure that the reaction rate was proportional to the enzyme concentration. For this purpose, 500 µl of tributyrin in 35 ml of lipolysis medium (without the addition of phosphatidylcholine) can be lipolyzed with 1.5 µl of trypsin. Enzyme activity must be greater than 900 U/mg. Lipolysis test: A formulation sample equivalent to 50 mg of SLFN was added to 36 ml of the lipolysis medium at 37°C. After stirring for 10 min, 4 ml of trypsin solution was added. Samples (approximately 1 ml) were taken periodically throughout the test (approximately 1 hour) and the samples were mixed with 4-bromophenylboronic acid (lipase inhibitor). The sample was centrifuged at 37°C, and the supernatant was taken and diluted for detection.

The sampling times include 10 and 0 min before enzyme addition (dispersion phase) and 5, 15, 30 and 60 mins after enzyme addition (digestion phase).

**Table 19. Results of lipolysis test on self-microemulsions**

| Formulations | Amount of dissolved drug (mg) | | | | | |
|---|---|---|---|---|---|---|
| | -10min | 0min | 5min | 15min | 30min | 60min |
| F72 | 48 | 49 | 48 | 48 | 49 | 48 |
| F73 | 50 | 49 | 50 | 50 | 48 | 49 |
| F74 | 52 | 52 | 52 | 50 | 51 | 51 |
| F75 | 49 | 49 | 48 | 40 | 33 | 21 |

It can be seen from the above results that the self-microemulsions added with the self-assembled construction unit materials can still maintain the synergistic regulation of molecular packing of sorafenib during the lipase digestion process, while the formulation F75 without adding a crystallization inhibitor showed a gradual increase in the molecular packing of sorafenib over time.

### Example 8 Donafenib preparations

Preparation process: The pharmaceutical compositions at the molecular level (SD) were prepared according to Example 2 (replacing sorafenib with donafenib in Example 2). The SD, self-assembled construction units, half of the formulation amount of a filler, a disintegrant, and half of the formulation amount of a lubricant were mixed well, subjected to dry granulation, added with the remaining half of the formulation amount of the filler, a glidant, and half of the formulation amount of the lubricant, mixed well, and pressed into tablets. The formulations of the tablet samples are shown in Tables 20 and 21.

**Table 20. Formulations of tablets sample (mg/tablet)**

| Formulations | Pharmaceutical compositions at the molecular level | | | Self-assembled construction units | | | |
|---|---|---|---|---|---|---|---|
| | Donafenib | VA6 4 | PVPK30 | HPMC E5 | HPMCAS H | HPMCAS L | Soluplus |
| F76 | 50 | / | 200 | / | / | / | / |
| F77 | 50 | 50 | 50 | 20 | 20 | / | / |
| F78 | 50 | 50 | / | 20 | 30 | / | / |
| F79 | 50 | 50 | / | / | / | 20 | 20 |

**Table 21. Formulations of tablets - amounts of other adjuvants (mg/ tablet)**

| Adjuvants | Filler | Disintegrant | Glidant | Lubricant |
|---|---|---|---|---|
| | SMCC | CCNa | SiO2 | MS |
| Amount | 121 | 22.5 | 15 | 3 |

Dissolution detection: paddle method, 900 mL of pH 6.8 phosphate buffer solution containing 0.1% sodium dodecyl sulfate (SDS), 100 rpm, 37°C. Sampling and sample treatment: 10 mL of the sample was taken and filtered with PES filter membrane (diameter of 25 mm, pore size of 0.45 µm). 8 mL of filtrate was discarded. 1-2 mL of the remaining filtrate was taken, and 10 mL of medium was added in the dissolution cup. The filtered sample was sent to analysis and detected after dilution. 1 tablet (200 mg) of reference list drug (RLD) and 4 tablets (200 mg) of the preparations of the present disclosure were used for detection. The dissolution detection was carried out by HPLC and the dissolution results are shown in the table below.

**Table 22. Dissolution results of tablet formulations**

| Formulations | Cumulative Dissolution (%) | | | | |
|---|---|---|---|---|---|
| | 5min | 15min | 30min | 60min | 120min |
| F76 | 39 | 47 | 35 | 26 | 21 |
| F77 | 88 | 86 | 65 | 51 | 39 |
| F78 | 87 | 89 | 68 | 54 | 39 |
| F79 | 82 | 88 | 67 | 54 | 43 |

F76 is a comparative example prepared with reference to Example 1 of Patent CN103301067 B.

Based on the above results, it can be seen that the dissolution of samples F77, F78 and F79 prepared by the present disclosure was obviously higher than that of F76.

### Example 9 PK test on beagles

Samples of Example F44 (50 mg, 4 tablets, T1), Example F33(50 mg, 4 tablets, T2), DB1 (50 mg, 4 tablets, T3) and reference preparation Nexavar (specification 200 mg, 1 tablet, R) were used to perform a PK test in fasting beagles.

Animals: Adult male beagle (8-11 kg), non-naive; divided into 3 groups, with 2 beagles in each group.

Route and frequency of administration: A single oral administration at 200 mg by gavage, fasting for more than 16 hours before administration, and free access to food 4 hours after administration; 4 days of a cleaning period, with 4 cycles of cross-administration.

Time points for blood sampling: 0, 1.5, 2, 2.5, 3, 4, 5, 6, 8, 12, 24, 48 h.

The drug level in blood was detected by LC/MS/MS method, and the pharmacokinetic parameters (AUC₀₋ₜ, AUC_{0-∞}, Cₘₐₓ, T_{1/2}, Tₘₐₓ and their arithmetic mean (± SD) and geometric mean) were calculated by Phoenix WinNonlin 7.0.

**Table 23. Results of PK test in beagles**

| Item | Cmax (ng/mL) | | | | AUClast (ng*h/mL) | | | |
|---|---|---|---|---|---|---|---|---|
| sample | R | T1 | T2 | T3 | R | T1 | T2 | T3 |
| Mean value | 2288 | 5521 | 5112 | 2816 | 13762 | 34297 | 33586 | 18005 |
| CV% | 56 | 31 | 36 | 45 | 52 | 32 | 34 | 44 |
| geometric mean | 1969 | 5327 | 4929 | 2758 | 12307 | 33767 | 32305 | 16009 |
| R test/reference | / | 2.7 | 2.5 | 1.4 | / | 2.7 | 2.6 | 1.3 |

Based on the above results, it can be seen that the bioavailability of F33 and F44 of the present disclosure was much higher than that of the reference preparation and the comparative example DB1.

This test confirmed that the bioavailability of sorafenib can be significantly improved by adding the self-assembled polymer materials.

### Example 10 PK test on healthy volunteers

The samples of Example F44 (50 mg, T1) and Example F33 (50 mg, T2) and the reference preparation Nexavar (specification 200 mg, R) were used for fasting PK test.

Subjects: 12 healthy Chinese men or women aged 18-65 years old (including 5 women)
Single center, randomized, three preparations, three cycles, triple cross-over design.

Route and frequency of administration: After the subjects fasted overnight for at least 10 hours, 1 tablet of the reference preparation and 2 tablets of each of T1 and T2 were administered the following morning on an empty stomach in 240 mL of aqueous solution. The cleaning period was 10 days.

Time points for blood sampling: before administration (0 h), 0.5 h, 1 h,1.5 h, 2 h, 2.5 h, 3 h, 3.5 h, 4 h, 6 h, 8 h, 12 h, 16 h, 24 h, 36 h, 48 h, 72 h, and 96 h after administration.

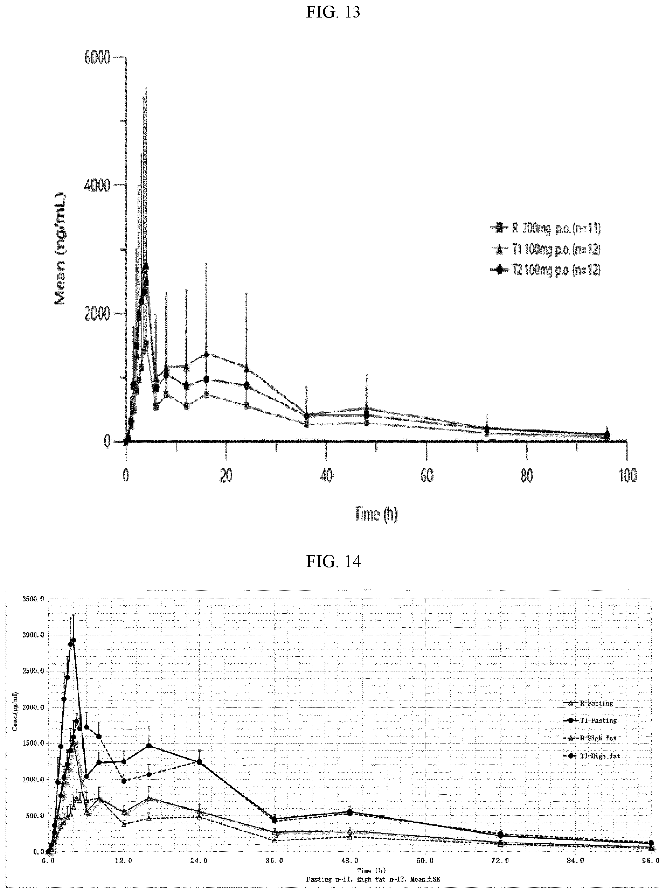

The drug level in blood was detected by LC/MS/MS method. The pharmacokinetic parameters (AUC₀₋ₜ, AUC_{0-∞}, Cₘₐₓ, T_{1/2}, Tₘₐₓ and their arithmetic mean (± SD) and geometric mean) were calculated by Phoenix WinNonlin 7.0, and the relative bioavailability was analyzed. The results are shown in the following Table 24, Table 25 and FIG. 13.

**Table 24. Results of PK test in humans - table I**

| Preparation | Item | Cmax (ng/mL) | AUClast (ng*h/mL) | AUCINF_obs (ng*h/mL) |
|---|---|---|---|---|
| R | Mean | 1534 | 32103 | 34117 |
| | SD | 987 | 19502 | 20777 |
| | CV% | 64.35 | 60.75 | 60.90 |
| T1 | Mean | 2859 | 59096 | 62409 |
| | SD | 1340 | 27714 | 29785 |
| | CV% | 46.87 | 46.90 | 47.73 |
| T2 | Mean | 2525 | 48502 | 52096 |
| | SD | 1427 | 23586 | 26112 |
| | CV% | 56.54 | 48.63 | 50.12 |

**Table 25. Results of PK test in humans - table II**

| Drug for test | Ln[Cmax(ng/mL)] | Ratio_%Ref | Tmax(h) | Ln[AUC_{0-∞}(ng*h/mL)] | Ratio_%Ref_ |
|---|---|---|---|---|---|
| T1-100 mg | 2555.57 | 210.77 | 3.71 | 55724.34 | 200.98 |
| T2-100 mg | 2135.40 | 200.98 | 3.72 | 45774.93 | 165.10 |
| RLD-200 mg | 1212.51 | -- | 4.23 | 27726.01 | -- |

Based on the above results, it can be seen that the administration of 50 mg of T1 was equivalent to 200 mg of the reference preparation Nexavar, and the administration of 60 mg of T2 was equivalent to 200 mg of the reference preparation Nexavar. Moreover, the Cmax and AUC variation of T1 and T2 preparations were lower than those of the reference preparation. This means that for future clinical application of products developed using the technical solutions provided by the present invention, 200 mg (Bid) can be used to replace Sorafenib Tosylate Tablets and generic drugs 800 mg (Bid), or Donafenib Tablets 400 mg (Bid).

### Example 11 PK test on healthy volunteers with high-fat diet

PK test of high-fat and high-calorie diet was carried out with the sample of Example F44 (50 mg, T1), and the reference preparation donafenib (specification 200 mg, R);
Subjects: 12 healthy Chinese men or women aged 18-65 years old (including 5 women)
Single center, randomized, open, after meal, single administration, two preparations, two cycles and double cross-over design.

Route and frequency of administration: Subjects were all fasted but with free drinking overnight (at least 10 h), and high-fat and high-calorie meals was eaten 30 min before the administration on the day of each cycle, and was finished within 30 min. The test drug was administered 30 min (±30 s) after the beginning of eating, and 100 mg of the test preparation of sorafenib tablets T1 or 200 mg of the reference preparation (R, Nexavar) were administered with 240 mL of water. The cleaning period per cycle was 10 days.

Time points for blood sampling: about 4 mL of cubital venous blood was collected before administration (0 h), and 1.0 h, 2.0 h, 2.5 h, 3.0 h, 3.5 h, 4.0 h, 4.5 h, 5.0 h, 6.0 h, 8.0 h, 12.0 h, 16.0 h, 24.0 h, 36.0 h, 48.0 h, 72.0 h and 96.0 h after administration.

The drug level in blood was detected by LC/MS/MS method. The pharmacokinetic parameters (AUC₀₋ₜ, AUC_{0-∞}, Cₘₐₓ, T_{1/2}, Tₘₐₓ and their arithmetic mean (± SD) and geometric mean) were calculated by Phoenix WinNonlin 7.0, and the relative bioavailability was analyzed. The results are shown in the following Table 26, Table 27 and FIG. 14.

**Table 26. Results of PK test in humans after high-fat meal - table I**

| Preparation | Item | Cmax (ng/mL) | AUClast (ng*h/mL) | AUCINF_obs (ng*h/mL) |
|---|---|---|---|---|
| R | Mean | 963 | 23191 | 24804 |
| | SD | 448 | 13848 | 15386 |
| | CV% | 46.51 | 59.71 | 62.03 |
| T1 | Mean | 2104 | 57693 | 62460 |
| | SD | 689 | 21605 | 27120 |
| | CV% | 32.74 | 37.45 | 43.42 |

**Table 27. Results of PK test in humans after high-fat meal - table II**

| Drug for test | Ln[Cmax(ng/mL)] | Ratio_%Ref | Tmax(h) | Ln[AUC_{0-∞}(ng*h/mL)] | Ratio_%Ref_ |
|---|---|---|---|---|---|
| T1-100 mg | 2009.4 | 231 | 5.5 | 58324.0 | 268 |
| RLD-200 mg | 870.3 | / | 7.8 | 21740.8 | / |

According to the instructions of Nexavar, when on a high-fat and high-calorie diet, the bioavailability of sorafenib is reduced by 29% compared with the fasting state. Therefore, the instructions of Nexavar stipulate that the administration should be performed on an empty stomach (without food (at least 1 hour before or 2 hours after a meal)). According to Example 9 and Example 10, the relative bioavailability of T1-100 mg and RLD-200 mg was 200.98% on an empty stomach, and the relative bioavailability was 268% on a high-fat and high-calorie diet. The ratio of Cₘₐₓ to AUC_{0-∞} of the test preparation T1 after/before meals was 1.1 and 1.3, respectively, while the AUC_{0-∞} of the reference preparation after meals decreased by about 30%. It can be seen that the preparation of the present disclosure can eliminate the impact of high-fat food on bioavailability and can achieve postprandial administration. Also, administration with food can significantly reduce gastrointestinal side effects such as nausea and diarrhea. Furthermore, according to Example 9 and Example 10, the CV of the preparations of the present disclosure was significantly smaller than that of the reference preparation Nexavar. This also helps to further improve the efficacy and reduce the side effects. Therefore, the preparations of the present disclosure have obvious clinical advantages and can bring significant benefits to patients.

According to the above data and analysis, the preparations of the present disclosure solve the problem of dissolution and absorption of sorafenib. Therefore, when the dosage needs to be increased due to drug resistance after long-term administration, the effective exposure can be conveniently maintained by dose titration, thus solving the drug resistance problem.

The description of the above examples is only used to help understand the method and its core idea of the present disclosure. It should be noted that those skilled in the art can make several improvements and modifications to the present disclosure without departing from the principles of the present disclosure, and these improvements and modifications also fall within the scope of the claims of the present disclosure.

The above description of the disclosed embodiments enables those skilled in the art to realize or use the present disclosure. Various modifications to these embodiments will be readily apparent to those skilled in the art, and the generic principles defined herein may be practiced in other embodiments without departing from the spirit or scope of the present disclosure. Thus, the present disclosure is not intended to be limited to the embodiments shown herein but is to be accorded the widest scope consistent with the principles and novel features disclosed herein.

## Claims

1. An oral preparation of sorafenib or donafenib with low administration dosage, comprising a pharmaceutical composition of sorafenib or donafenib at molecular level, a self-assembled polymer material and an additional pharmaceutically acceptable adjuvant, wherein the pharmaceutical composition of sorafenib or donafenib at molecular level is a solid dispersant, solid solution, polymer nanoparticle, self-microemulsion, nanoemulsion at molecular level or a mixture at molecular level;
wherein, the self-assembled polymer material is selected from the group consisting of povidone + hydroxypropyl cellulose, povidone with low viscosity + povidone with high viscosity, povidone + copovidone, povidone + polyvinylcaprolactam-polyvinyl acetate-polyethylene glycol graft copolymer, povidone + hydroxypropyl methylcellulose acetate succinate, povidone + hydroxypropyl methylcellulose, povidone + cellulose acetate, povidone + croscarmellose sodium, povidone + polyethylene glycol, povidone + low-substituted hydroxypropyl cellulose, povidone + hydroxypropyl methylcellulose phthalate, povidone + methacrylic acid-ethylacrylate copolymer, povidone + methacrylic acid-methacrylate copolymer, copovidone + hydroxypropyl cellulose, copovidone + polyvinylcaprolactam-polyvinyl acetate-polyethylene glycol graft copolymer, copovidone + hydroxypropyl methylcellulose acetate succinate, copovidone + hydroxypropyl methylcellulose, copovidone + cellulose acetate, copovidone + croscarmellose sodium or croscarmellose calcium, copovidone + polyethylene glycol, copovidone + low-substituted hydroxypropyl cellulose, copovidone + hydroxypropyl methylcellulose phthalate, copovidone + methacrylic acid-ethylacrylate copolymer, copovidone + methacrylic acid-methacrylate copolymer, polyvinylcaprolactam-polyvinyl acetate-polyethylene glycol graft copolymer + hydroxypropyl methylcellulose acetate succinate, polyvinylcaprolactam-polyvinyl acetate-polyethylene glycol graft copolymer + hydroxypropyl methylcellulose, polyvinylcaprolactam-polyvinyl acetate-polyethylene glycol graft copolymer + hydroxypropyl cellulose, polyvinylcaprolactam-polyvinyl acetate-polyethylene glycol graft copolymer + low-substituted hydroxypropyl cellulose, polyvinylcaprolactam-polyvinyl acetate-polyethylene glycol graft copolymer + croscarmellose sodium or croscarmellose calcium, polyvinylcaprolactam-polyvinyl acetate-polyethylene glycol graft copolymer + polyethylene glycol, polyvinylcaprolactam-polyvinyl acetate-polyethylene glycol graft copolymer + hydroxypropyl methylcellulose phthalate, polyvinylcaprolactam-polyvinyl acetate-polyethylene glycol graft copolymer + methacrylic acid-ethylacrylate copolymer, and polyvinylcaprolactam-polyvinyl acetate-polyethylene glycol graft copolymer + methacrylic acid-methacrylate copolymer.

2. The oral preparation of sorafenib or donafenib according to claim 1, wherein the self-assembled polymer material accounts for 10-50wt% of the pharmaceutical composition of sorafenib or donafenib at molecular level.

3. The oral preparation of sorafenib or donafenib according to claim 1, wherein the hydroxypropyl methylcellulose is HPMC E5 or HPMCK100LV; the self-assembled polymer material is a self-assembled polymer material compounded with one of povidone, copovidone, polyvinylcaprolactam-polyvinyl acetate-polyethylene glycol graft copolymer and hydroxypropyl methylcellulose acetate succinate H type with strong hydrophobicity, and HPMC E5, at a compounding ratio of (1-10): 1; or the self-assembled polymer material is a self-assembled polymer material compounded with one of polyvinylcaprolactam-polyvinyl acetate-polyethylene glycol graft copolymer and hydroxypropyl methylcellulose acetate succinate H type with strong hydrophobicity, and HPMCK100LV, at a compounding ratio of (1-10): 1.

4. The oral preparation of sorafenib or donafenib according to claim 1 or 3, wherein the hydroxypropyl methylcellulose acetate succinate has an acetyl substitution amount of 6-16% and a succinyl substitution amount of 4-16%.

5. The oral preparation of sorafenib or donafenib according to claim 1 or 3, wherein the povidone has an average molecular weight of 34,000 to 1,300,000, or the povidone is compounded with povidone with an average molecular weight of 34,000 and povidone with an average molecular weight of 1,300,000 at a ratio of 1: 1-1:0.1.

6. The oral preparation of sorafenib or donafenib according to claim 1, wherein a unit dose of the oral preparation contains 20-69 mg of sorafenib or donafenib.

7. The oral preparation of sorafenib or donafenib according to claim 1, wherein the pharmaceutical composition of sorafenib or donafenib at molecular level comprises sorafenib or donafenib, or a salt, hydrate, or solvate thereof, or a hydrates or solvate of a salt thereof, and a carrier; the carrier is selected from the group consisting of copovidone, povidone, polyvinylcaprolactam-polyvinyl acetate-polyethylene glycol graft copolymer, povidone + hydroxypropyl methylcellulose acetate succinate, povidone + hydroxypropyl methylcellulose, povidone with low viscosity + povidone with high viscosity, povidone + copovidone, povidone + polyvinylcaprolactam-polyvinyl acetate-polyethylene glycol graft copolymer, copovidone + hydroxypropyl methylcellulose acetate succinate, copovidone + polyvinylcaprolactam-polyvinyl acetate-polyethylene glycol graft copolymer, copovidone + hydroxypropyl methylcellulose, copovidone + polyethylene glycol, copovidone + hydroxypropyl cellulose, copovidone + cellulose acetate, polyvinylcaprolactam-polyvinyl acetate-polyethylene glycol graft copolymer + hydroxypropyl methylcellulose, polyvinylcaprolactam-polyvinyl acetate-polyethylene glycol graft copolymer + hydroxypropyl methylcellulose acetate succinate, polyvinylcaprolactam-polyvinyl acetate-polyethylene glycol graft copolymer + povidone, and polyvinylcaprolactam-polyvinyl acetate-polyethylene glycol graft copolymer + copovidone.

8. The oral preparation of sorafenib or donafenib according to claim 7, wherein the sorafenib or donafenib, or a salt, hydrate, or solvate thereof, or a hydrates or solvate of a salt thereof accounts for 30-60wt% of the pharmaceutical composition of sorafenib or donefenib at molecular level.

9. The oral preparation of sorafenib or donafenib according to claim 1, wherein a dosage form of the oral preparation of sorafenib or donafenib is tablet, suspension, granule or capsule.

10. The oral preparation of sorafenib or donafenib according to claim 9, wherein the tablet comprises an adjuvant selected from the group consisting of a filler, disintegrant, glidant and lubricant; the filler is selected from the group consisting of microcrystalline cellulose, silicified microcrystalline cellulose, and a mixture thereof; the disintegrant is croscarmellose sodium; the glidant is silicon dioxide; and the lubricant is selected from the group consisting of magnesium stearate, sodium stearyl fumarate, and a mixture thereof.

11. The oral preparation of sorafenib or donafenib according to claim 10, wherein a content of the filler is 30-45wt% of the tablet, a content of the disintegrant is 5-15wt% of the tablet, a content of the glidant is 3-5wt% of the tablet, and a content of the lubricant is 1-5wt% of the tablet.

12. The oral preparation of sorafenib or donafenib according to any one of claims 1-11, wherein the oral preparation of sorafenib or donafenib is administered with or without food.

13. Use of the oral preparation of sorafenib or donafenib according to any one of claims 1-12 in the manufacture of a medicament for treating or alleviating hepatocellular carcinoma, breast cancer, primary malignant liver tumor, cholangiocarcinoma, renal cell carcinoma, recurrent or refractory solid tumor, central nervous solid tumor, metastatic non-small cell lung cancer, unresectable hepatocellular carcinoma, thyroid cancer, pancreatic cancer, adenoid cystic carcinoma of the salivary gland, non-differentiated thyroid carcinoma, refractory differentiated thyroid cancer, adenoid cystic carcinoma, advanced endometrial cancer, anaplastic thyroid cancer, rectal cancer, colon cancer, unresectable stage III or IV melanoma, leukemia, acute myeloid leukemia, all solid tumors or advanced solid tumors.
